# EUROPEAN PATENT SPECIFICATION

(11) **EP 1 218 019 B1**
(45) Date of publication and mention of the grant of the patent: **08.03.2006**
(21) Application number: 00965106.8
(22) Date of filing: 18.09.2000
(51) Int. Cl.: A61K 35/76, A61P 35/00, C12N 7/00, C12N 7/02, C12N 15/47, A61K 38/21

(54) **ONCOLYTIC VESICULAR STOMATITIS VIRUS**
ONKOLYTISCHES VESICULAR STOMATITIS VIRUS
VESICULAR STOMATITIS VIRUS ONCOLYTIQUE

(30) Priority: 17.09.1999 US 397873
(43) Date of publication of application: 03.07.2002
(60) Divisional application: 05025974.6
(73) Proprietor: Wellstat Biologics Corporation, Gaithersburg, MD 20878 (US)
(72) Inventor: BELL, John, Cameron, Ottawa, Ontario K1G 0K8 (CA); SONENBERG, Nahum, Montreal, Quebec H4W 2S9 (CA); STOJDL, David, Francis, Ottawa, Ontario K2P 0H6 (CA); BROWN, Earl, Garnet, Nepean, Ontario K2E 5Y6 (CA); ATKINS, Harold, Lawrence, Orleans, Ontario K1C 8E3 (CA); MARIUS, Ricardo, Marcellus, Ottawa, Ontario K1G 1R2 (CA); LICHTY, Brian, Dennis, Russell, Ontario K4R 1E5 (CA); KNOWLES, Shane, Brendan, Ottawa, Ontario K1N 5C3 (CA)
(74) Representative: MacLean, Martin Robert
(86) International application number: PCT/US2000/025510
(87) International publication number: WO 2001/019380

(56) References cited:
- WO-A-00/62735
- WO-A-96/34625
- WO-A-99/18799
- WO-A-99/45783
- NICKELS MICHAEL S ET AL: "Identification of an amino acid change that affects N protein function in vesicular stomatitis virus." JOURNAL OF GENERAL VIROLOGY, vol. 75, no. 12, 1994, pages 3591-3595, XP002162485 ISSN: 0022-1317
- SCHUBERT M ET AL: "PRIMARY STRUCTURE OF THE VESICULAR STOMATITIS VIRUS POLYMERASE L GENE EVIDENCE FOR A HIGH FREQUENCY OF MUTATIONS" JOURNAL OF VIROLOGY, vol. 51, no. 2, 1984, pages 505-514, XP000982855 ISSN: 0022-538X
- FRANCOEUR A M ET AL: "THE ISOLATION OF INTERFERON-INDUCING MUTANTS OF VESICULAR STOMATITIS VIRUS WITH ALTERED VIRAL P FUNCTION FOR THE INHIBITION OF TOTAL PROTEIN SYNTHESIS" VIROLOGY, vol. 160, no. 1, 1987, pages 236-245, XP000982787 ISSN: 0042-6822 cited in the application
- BALACHANDRAN SIDDHARTH ET AL: "Activation of the dsRNA-dependent protein kinase, PKR, induces apoptosis through FADD-mediated death signaling" EMBO JOURNAL,OXFORD UNIVERSITY PRESS, SURREY,GB, vol. 17, no. 23, 1 December 1998 (1998-12-01), pages 6888-6902, XP002159409 ISSN: 0261-4189
- DURBIN J E ET AL: "TARGETED DISRUPTION OF THE MOUSE STAT1 GENE RESULTS IN COMPROMISED INNATE IMMUNITY TO VIRAL DISEASE" CELL,CELL PRESS, CAMBRIDGE, NA,US, vol. 84, 9 February 1996 (1996-02-09), pages 443-450, XP000941705 ISSN: 0092-8674
- STOJDL DAVID F ET AL: "Exploiting tumor-specific defects in the interferon pathway with a previously unknown oncolytic virus." NATURE MEDICINE, vol. 6, no. 7, July 2000 (2000-07), pages 821-825, XP002162486 ISSN: 1078-8956
- BALACHANDRAN SIDDHARTH ET AL: "Vesicular stomatitis virus (VSV) therapy of tumors." IUBMB LIFE, vol. 50, no. 2, August 2000 (2000-08), pages 135-138, XP000982784 ISSN: 1521-6543

## Description

The present invention relates to a novel cancer therapeutic. More specifically, this invention relates to viruses that selectively infect and inhibit tumour cell growth.

### BACKGROUND OF THE INVENTION

The use of oncolytic bacteria, or compositions of oncolytic bacterias, for combatting neoplasms in humans and animals is known. For example EP 564 121, GB 1,587,244 and U.S. 3,192,116 disclose the use of non-pathogenic bacteria that result in the liquification and lysis of tumours in vertebrates. However in many instances, for example with the use of *Clostridium*, the tumours are only partially destroyed, and tumour regrowth may still occur. To ensure control of tumour growth the administration of bacteria, followed by chemotherapeutic drugs, for example 5-fluorodeoxyuridine or alkylating agents, has been suggested (e.g. GB 1,069,144).

Several viruses have also been shown to exhibit tumoricidal properties, for example parvovirus H-1 (Dupressoir et al., 1996. Cancer Res, 49:3203-3208). Newcastle disease virus (Reichand et al., 1992. J. Surg. Res, 52:448-453) or retroviral vectors containing drug susceptibility genes (Takamiya et al., 1993. J. Neurosurg, 79:104-110). WO97/26904 and WO96/03997 disclose a mutant herpes simplex virus (HSV-1761) that inhibits tumour cell growth. Administration of HSV-1716 comprising a 759 base pair deletion in each copy of γ34.5 of the long repeat region (R_{L}) to tumour cells kills these cells. However, this virus is specific for neuronal cells as HSV is known to selectively inhabit the neuronal system. Furthermore, the use of common human pathogens as an oncolytic virus is limited as it is likely that the general population has been infected and acquired an immune response to such viruses. A preexisting immune response to a viral strain similar to the one used as a therapeutic agent in the treatment of a cancer may attenuate the effectiveness of the virus as therapeutic agent.

Other virus strains have reported oncolytic activity. The ONYX-015 human adenovirus (produced by ONYX pharmaceuticals) is believed to replicate preferentially in p53 negative tumour cells. This virus shows promise in clinical trials with head and neck cancer patients (Kim, D., T. et al., Nat Med, 1998. 4:1341-1342). Reovirus type 3 is being developed by Oncolytic Biotech as a cancer therapeutic, which preferentially grows in PKR -/- cells (Yin, H.S.,. J Virol Methods, 1997. 67:93-101; Strong, J.E. and P.W. Lee,. J Virol. 1996. 70:612-616; Strong, J.E., et al., Virology, 1993. 197:405-411; Minuk, G.Y., et al., J Hepatol, 1987. 5:8-13; Rozee, K.R., et al., Appl Environ Microbiol, 1978. 35:297-300). Reovirus, type III exhibited enhanced replication properties in cells which expressed the mutatnt ras oncogene (Coffey, M.C., et al., Science, 1998. 282:1332-1334; Strong, J.E., et al., Embo J, 1998. 17:3351-1362). Mundschau and Faller (Mundschau, L.J. and D.V. Faller, J Biol Chem, 1992. 267:23092-23098) have shown that the ras oncogene product activated an inhibitor of PKR, and this coupled with the observation that the PKR chemical inhibitor 2-aminopurine increased the growth of Reo type III in normal cells implicates PKR is a critical regulator of the growth of reovirus.

W0 99/04026 teaches the use of VSV as a vector in gene therapy for the expression of a wide range of products including antibodies, immunogens, toxins, etc. for the treatment of a variety of disease disorders.

WO 99/45783 describes producer cells for replication selective viruses in the treatment of malignancy.

WO 96/34625 describes recombinant vesiculoviruses and their uses.

Interferons are circulating factors which bind to cell surface receptors activating a signalling cascade ultimately leading to a number of biological responses.

Two of the outcomes of interferon signalling are tightly linked: (1) an antiviral response and (2) induction of growth inhibitory and/or apoptotic signals.

U.S. 4,806,347 discloses the use of γ Interferon and a fragment of INF-γ (known asΔ4α2) against human tumour cells.

WO 99/18799 reports the cytotoxic activity of Newcastle Disease Virus (NDV) and Sindbis virus towards several human cancer cells. However, both viruses demonstrated selectivity in their cytotoxic activity towards tumor cells.

WO 99/18799 discloses that interferon addition to normal cells renders these cells resistant to NDV, yet, this effect was not observed with interferon-treated tumor cells which continued to exhibit NDV-induced sensitivity. WO 99/18799 also discloses the cytotoxic activity of VSV cells against KB cells (head and neck carcinoma) and HT 1080 (Fibrosarcoma), and alleviation of cytotoxicity in normal and tumor cells, by VSV, in the presence of interferon. No other cell types were tested against VSV cytotoxic activity.

Certain mutant strains of VSV have been reported. Stanners, et al., Virology (1987) 160(1):255-8. Francoeur, et al., Virology (1987) 160(1):236-45. Stanners, et al., J. Gen. Virol. (1975) 29(3):281-96. Stanners, et al., Cell (1977) 11(2):273-81.

The present invention relates to viral formulations that are useful in the treatment of diseases and cancers, preferably leukaemia. Such formulations may also comprise an oncolytic VSV strain and a chemical agent, for example a cytokine which confers to normal cells, resistance to viral infection, but leaves diseased or cancerous cells susceptible to viral infection and lysis.

It is an object of the invention to overcome disadvantages of the prior art.

The above object is met by the combinations of features of the main claims, the sub-claims disclose further advantageous embodiments of the invention.

### SUMMARY OF THE INVENTION

The present invention relates to a novel cancer therapeutic. More specifically, this invention relates to viruses that selectively infect and inhibit tumour cell growth.

### According to the present invention there is provided the use of a virus in the manufacture of a medicament for reducing the viability of a tumor cell, wherein the virus is an attenuated strain of vesicular stomatitis virus and wherein the tumor cell is a hematopoietic cancer cell, a melanoma, a sarcoma, a neuroendocrine tumor, a lung carcinoma or a colon carcinoma

This invention is also directed to reducing the viability of a tumour cell by administration of VSV to the tumour cell, wherein the VSV is unable to inactivate PKR activity within a host cell.

The present invention also pertains to reducing the viability of a tumour cell within a population of cells by administration of VSV to the population of cells, wherein the VSV is selectively able to infect and kill the tumour cell. Preferably the VSV is unable to inactivate PKR activity in a host cell.

This invention also relates to reducing the viability of a tumour cell within a population wherein the population of cells is treated with interferon prior to administration of the VSV.

The present invention is directed to a mutant VSV, characterized in that the mutant VSV grows poorly in interferon-responsive cells. Such strains are also referred to herein as attenuated strains of VSV, or VSV strains that grow poorly in interferon-responsive cells. They can be identified by their producing smaller plaques in monolayers of interferon-responsive cells than in interferon-nonresponsive cells, as described below. Attenuated VSV strains can also be identified by their having a higher LD50 when administered intranasally to PKR+/- mice as compared to WT Indiana, in the assay described below.

The present invention is also directed to isolated nucleic acid molecules (DNA or RNA) having a sequence coding for mutant VSV proteins and sequences complementary thereto. Such nucleic acid molecules can be used in the preparation of a recombinant VSV or as a DNA vaccine.

There are several advantages for the use of a virus as described herein as a therapeutic virus over other viruses:
- Rhabdoviruses are not common human pathogens. For example, VSV is found mostly in insects, rodents and domestic farm animals, and therefore a large proportion of individuals will not have been infected or immunized to VSV infection. On the other hand, Adenovirus or Reovirus are human pathogens and most of the general population have been infected and acquired an immune response to both of these viruses. A preexisting immune response to a viral strain similar to the one used as a therapeutic agent in the treatment of a cancer may attenuate the effectiveness of the virus as therapeutic agent;
- VSV replicates much more quickly than either Adenovirus or Reovirus, and can be readily concentrated to high titres. Production of high titre virus preparations is a significant limitation of other potential viral therapeutic strains;
- VSV is simple virus comprising only five genes, easily amenable to genetic manipulation. No such system is currently available for Reovirus;
- Cellular infection by VSV is highly responsive to additional chemical agents such as interferon, a feature which enhances its therapeutic value.
- VSV has a broad host range and is capable of infecting most types of human cells, whereas other viruses are more limited in regard to the types of cells they may infect.
- VSV is a RNA virus and spends its entire lifecyle in the cytoplasm. Therefore it involves less danger of unwanted integration into the genome of a patient.
Collectively, these VSV attributes provide significant advantages over the use of the other viruses known to exhibit oncolytic activity.

### BRIEF DESCRIPTION OF THE DRAWINGS

These and other features of the invention will become more apparent from the following description in which reference is made to the appended drawings wherein:
**FIGURE 1** shows a general schematic of the interferon cascade.
**FIGURE 2** shows the effect of VSV on normal human fibroblasts, human melanoma cell line SK-MEL3, LNCaP a prostate cancer cell line, and the ovarian carcinoma cell A2780 in the presence and absence of interferon as determined by a modified cpe assay. Monolayers of cells were infected at an moi of 0.1 pfu in a 12 well plate. At time 0 and every 12 hours subsequent up to 48 hours, one well of infected cells was fixed with 0.5 ml Leukostat fixative for 2 minutes. At the end of the experiment monolayers were stained with Leukostat stains.
**FIGURE 3** shows the cytopathic effect of VSV in normal fibroblasts cells (Figure 3 (a)), and tumour cell lines, including ovarian tumour cells (Figure 3 (b)) and KB tumour cells (Figure 3 (c)).
**FIGURE 4** shows the effect of VSV on normal human fibroblasts co-cultured with 293T tumour cells over a period of 24 hours. Co-cultures were infected at an moi of 0.1 pfu/cell and the infection allowed to proceed in the presence (IFN+) or absence (IFN-) of interferon. Cultures were stained with antibodies to large T antigen (red nuclei) to detect the 293T cells and with DAPI (blue nuclei) which stains all cell types.
**FIGURE 5** shows the effect of VSV *in vivo* on tumors implanted within nude mice. Human melanoma cells were implanted within nude mice and either mock injected (VSV(-)), injected with wild type VSV (data not presented), or injected with additional melanoma cells infected in vitro with VSV for one hour prior to injection into the tumour site VSV(+)). Size of the tumors were determined over a 7 day period.
**FIGURE 6** shows ulcers formed on a tumor produced within a nude mouse as described in Figure 5.
**FIGURE 7**: VSV and VSV infected cells inhibit growth of human melanoma xenografts in nude mice.
**FIGURES 8A and 8B:** PKR -/- mice are acutely sensitive to intranasal VSV infection and demonstrate a deficiency in IFN mediated resistance.
**FIGURE 9:** Interferon can protect xenograft bearing nude mice during VSV treatment.
**FIGURES 10A and 10B:** Virus production from tumour cells and normal cells infected with wild type Indiana and various mutant VSV strains.
**FIGURE 11:** Malignant cells are rapidly killed following VSV (WT Indiana) infection and are not protected by IFN-α.
**FIGURE 12**: VSV induced cytopathic effect visible in human melanoma cells but not in primary human cells with or without IFN-α.
**FIGURE 13**: Efficacy of a single intravenous dose of mutant VSV in treating human melanoma xenografts in nude mice.
**FIGURE 14:** N Protein cDNA sequence of wild type and mutant VSVs.
**FIGURE 15:** N Protein amino acid sequence of wild type and mutant VSVs.
**FIGURE 16:** P Protein cDNA sequence of wild type and mutant VSVs.
**FIGURE 17:** P Protein amino acid sequence of wild type and mutant VSVs.
**FIGURE 18:** M Protein cDNA sequence of wild type and mutant VSVs.
**FIGURE 19**: M Protein amino acid sequence of wild type and mutant VSVs.
**FIGURE 20**: G Protein cDNA sequence of wild type and mutant VSVs.
**FIGURE 21**: G Protein amino acid sequence of wild type and mutant VSVs.
**FIGURE 22:** L Protein cDNA sequence of wild type and mutant VSVs.
**FIGURE 23**: L Protein amino acid sequence of wild type and mutant VSVs.

### DESCRIPTION OF PREFERRED EMBODIMENTS

The present invention relates to a novel cancer therapeutic. More specifically, this invention relates to viruses that selectively infect and inhibit tumour cell growth.

The following description is of a preferred embodiment by way of example only and without limitation to the combination of features necessary for carrying the invention into effect.

Cancer cells gain a survival advantage over their normal counterparts by acquiring mutations in growth inhibitory or apoptotic pathways and, in the case of interferons, would do so at the expense of critical antiviral defence mechanisms. As tumour cells gain a significant growth advantage by mutating interferon response genes, they will be more susceptible to virus infection.

By "reducing the viability" of a tumour cell it is meant either killing the tumour cell or limiting its growth for a period of time.

By "not a common human pathogen" it is meant a virus that is found mostly in non-human hosts, for example, but not limited to insects, rodents, and farm animals. Such viruses are not typically found within the general human population.

As used herein Mutant I, Mutant 1, Mut 1 and M1 refer to attenuated mutant strain T1026. Mutants II, III, IV and V (and variant nomenclature analogous to Mutant I) refer to attenuated mutants T1026R, TP3, TP6 and G31, respectively.

The novel cancer therapeutic of the present invention incorporates the use of at least one oncolytic virus that selectively targets tumour cells and leads to their destruction. Preferably the oncolytic virus is a Vesicular stomatitis virus (VSV), for example the Indiana strain, or other strains, or a derivative thereof. By a derivative of VSV, it is meant a VSV virus obtained by either selecting the virus under different growth conditions, or one that has been subjected to a range of selection pressures, or one that has been genetically modified using recombinant techniques known within the art. For example, which are not to be considered limiting in any manner, a derivative of VSV may include a mutant VSV selected following infection on a human cell that has been treated with interferon as described herein, or a VSV that displays an affinity tag useful for affinity purification.

The effectiveness of oncolytic virus suppression of tumour cell growth in part resides in the differential susceptibility of tumour cells, compared to normal cells, to viral infection. Without wishing to be bound by theory, the differential susceptibility may in part be due to the down regulation or inactivation of factors within a cell that otherwise function to protect the cell from tumorous growth and virus infection. Examples of factors that when inactivated result in tumorous cell growth, and that are also involved in mediating virus infection include but are not limited to PKR (double stranded RNA dependent kinase) and PML (Promyelocytic Leukemia gene), however, it is to be understood that other factors may also play a role.

The down regulation or inactivation of PKR, through a variety of mechanisms including but not limited to PKR-related mediators, is known to be associated with tumour cell growth, while normal cells exhibit active PKR. Furthermore, wild type cells exposed to viral infection exhibit elevated PKR expression which results in the suppression of viral replication, while cells that exhibit reduced, or no, PKR activity are susceptible to viral attack and exhibit cancerous growth. Similarly, the PML gene product functions as a tumour suppressor and it is also known to suppress viral replication.

By "differential susceptibility", it is meant a property associated with a cell that results in both tumour cell growth and the inability of the cell to suppress viral replication. Cells exhibiting differential susceptibility are preferred candidates for treatment of tumorous cell growth using the cancer therapeutic of the present invention. This differential susceptibility may be accentuated through the addition of one or more chemical agents prior to or during treatment of the tumour cell. Preferably, this chemical agent increases the resistance of a wild-type cell to viral infection, but has little or no effect on the response of a tumour cell to viral infection. An example, which is not to be considered limiting in any manner, of such a chemical agent is interferon.

By "PKR" it is meant a serine/threonine kinase that exhibits multiple functions including roles in the control of mRNA translation and gene transcription (1,2). The kinase harbors two double-stranded RNA dsRNA binding motifs in its amino terminal regulatory half and catalytic kinase domain in its carboxyl tail. Binding of dsRNA to the amino terminus induces a conformational change in the enzyme revealing and activating the catalytic kinase domain. The expression of PKR is induced by several PKR-mediators, including but not limited to, interferon.

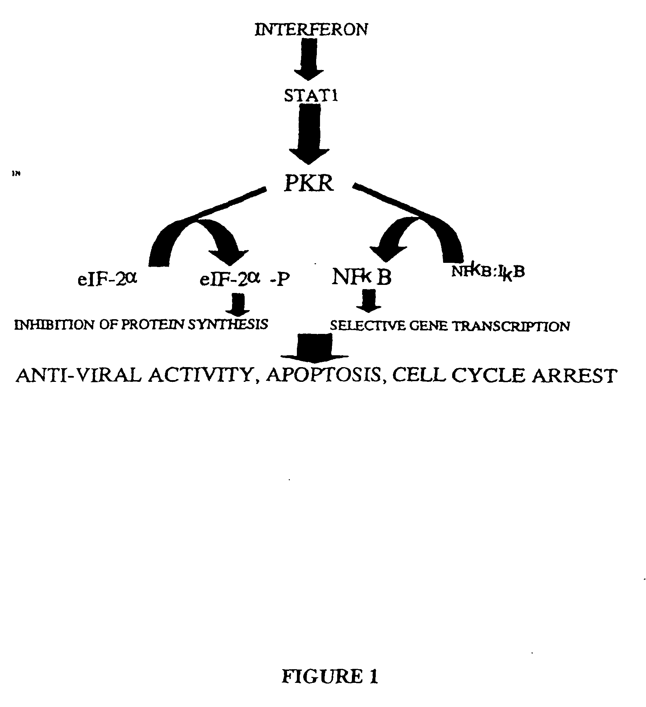

By "PKR-mediator" it is meant proteins or compounds that directly, or indirectly affect PKR activity either at the gene or protein level and include both PKR-activators and PKR-inhibitors. Examples of PKR-activators include, but are not limited to STAT1 (see Figure 1), Interferon regulatory factor (IRF-1), and interferon. Examples of PKR-inhibitors, include, but are not limited to, VA RNAs, p58(IPK), factors associated with the Ras pathway, the ribosomal protein L18, or proteases that degrade PKR protein. PKR activity may also be mediated through mutations to the gene encoding PKR, or to the regulatory region that drives the expression of *PKR*. These mutations may either increase or decrease PKR activity. Mutations to PKR that reduce PKR activity include, but are not limited to, the loss of dsRNA binding ability of PKR, or mutations that result in negative catalytic mutants. Mutations that increase PKR activity include, but are not limited to over-expression of PKR, or mutations that resulted in a more active PKR protein.

PKR regulates translation through the phosphorylation of eIF-2α, a factor involved in the initiation of protein translation. Once phosphorylated, eIF-2α-GDP, forms an inactive complex with eIF-2B resulting in a rapid inhibition of protein synthesis. PKR impinges on gene transcription indirectly via activation of NFκB. This activation appears to be carried out by PKR phosphorylation of an IκB kinase (3) which in turn phosphorylates IκB leading to its targeted destruction.

### PKR Antiviral activity

Infection of a cell by many distinct virus types leads to the formation of dsRNA (e.g. as replicative intermediates) resulting in the activation of PKR and its subsequent downstream effectors (see Figure 1). In particular, protein synthesis is rapidly terminated and an apoptotic cascade is initiated (4,5). As a result of the activation of PKR, the production of new virions is curtailed and the spread of virus through the organism is limited. Der et al (12) report a requirement for PKR in the induction of cellular apoptosis in response to a variety of stress inducers.

Without being bound by theory, it is possible that malignancies arise as a result of multiple mutations in genes that control cell proliferation and apoptosis. PKR's role in regulating protein synthesis coupled with its antiproliferative and pro-apoptotic properties make it a target for oncogenic mutations, which directly or indirectly affect its activity.

As described in more detail in the examples an initial screen of several viruses using PKR -/- animals indicated that PKR null animals are susceptible to infection by Vesicular stomatitis virus (VSV). Similar results were obtained *in vitro,* where VSV infection proceeded more rapidly in PKR-/- fibroblasts, when compared to infection in PKR +/+ fibroblasts. These results demonstrate that PKR is required by mammalian cells to resist infections by VSV. Furthermore, certain cell lines, for example, but not limited to primary human bone marrow, were resistant to VSV infection, while leukemia cell lines were susceptible to VSV infection.

It is contemplated that viruses related to VSV, or other viruses that exhibit similar mechanisms of viral infection can be identified that exhibit the property of selectively infecting cells with reduced or no PKR activity. One of skill in the art can readily screen other viruses using the methods as described herein, for their ability to reduce the viability of cells, or kill cells lacking PKR activity, or PKR-/- cells, PKR - /- animals, or both PKR-/- cells and animals.

As indicated in the examples below, pretreatment of cells with interferon reduces virus infectivity by several orders of magnitude. Without wishing to be bound by theory, the addition of interferon may upregulate PKR expression resulting in this increased resistance to viral infection.

Because of its potent antiviral activity, viruses have evolved strategies to circumvent PKR. For example, HIV and Hepatitis C encode proteins dedicated to the binding and inactivation of PKR (6,7). Adenovirus encodes small RNA molecules (VA RNAs) which bind to but do not activate PKR (8). Influenza virus usurps a cellular protein p58(IPK) to inhibit PKR while polio virus initiates the proteolytic degradation of PKR (9,10). Large T antigen of SV-40 appears to function downstream of eIF-2α to promote protein translation even in the presence of activated PKR (10).

### PKR and Tumour Suppression

Expression of dominant negative PKR catalytic mutants in NIH 3T3 cells leads to their malignant transformation and facilitates their growth as tumours in nude mouse models (13,14). A similar phenomena has been observed using PKR mutants which have lost dsRNA binding activity. Induced expression of PKR in *S*. *cerevisiae* leads to growth arrest in the yeast-a phenomena that can be reversed by co-expression of a non-phosphorylatable version of eIF-2α. Therefore, PKR has anti-proliferative activity and functions as a tumour suppressor.

There are several lines of evidence that PKR is inactivated, absent or reduced in expression in a broad spectrum of human malignancies:
- Oncogenic Ras mutations occur in about 30% of all human tumours while mutations in upstream Ras activators (ie EGF receptor, Neu receptor, PDGF receptor) are even more common. Mundschau and Faller (15,16) have described an oncogenic Ras induced PKR inhibitor. Furthermore, Strong et al (17) demonstrated that activation of the Ras pathway results in down regulation of PKR activity.
- The ribosomal protein L18 is overexpressed in primary colorectal cancer tissues and has recently been shown to bind to and inactivate PKR (18).
- Patients with 5q translocations exhibit diminished PKR expression (19-21). Interferon regulatory factor 1 (IRF-1) is a transcription factor with tumour suppressor activity, which maps to the human chromosomal region 5q. PKR gene transcription is regulated in part by IRF-1.
- Human PKR maps to 2p21-22 and has been recently identified as the site of translocation in a case of acute myelogenous leukemia .
- In biopsies from poorly differentiated, highly malignant tumours, PKR protein was present at very low levels or was undetectable (23-25).

### STAT1

STAT1 is an essential mediator of the interferon pathway and its activation results in an upregulation of PKR mRNA and protein (see figure 1).

There is marked deficiency in the level/activity of STAT1 protein in interferon resistant melanoma cell lines and primary melanoma biopsy material (26), in a variety of human tumour cell lines including a myeloid leukemia, cervical carcinomas, ovarian cancer, and a lung carcinoma (27), and in a gastric adenocarcinoma (28,29). Furthermore, cutaneous T cell lymphoma (CTCL) is a malignancy which in general is responsive to interferon (however frequently clinical resistance arises in a substantial portion of cases). Sun et al (30) have reported that STAT1 protein is absent in a CTCL cell line suggesting that development of clinical resistance to interferon may arise due to STAT1 mutations.

### PML: Promyelocytic Leukemia Gene

PML is an interferon induced gene that normally functions as a tumour suppressor and a key regulator of Fas, TNFα and interferon induced apoptosis. Recently, Chelbi-Alix et al have shown that another normal function of the PML gene product is to suppress virus replication. The PML-RAR fusion protein functions as a dominant negative inhibitor of interferon induced apoptosis and we would predict will also make APL cells preferentially susceptible to virus infection.

Down regulation of PKR protein or activity occurs in a broad spectrum of human malignancies. While cancer cells have attained a growth advantage and unbridled protein translation capacity by eliminating PKR or PKR-mediators, these cells have simultaneously eliminated one of the cell's primary and potent antiviral defence mechanisms. Therefore, tumour cells with reduced PKR activity will be more susceptible to infection than their normal counterparts. As indicated above, other components (e.g. STAT1 and PML) of the interferon pathway are frequently mutated in human malignancies, and loss of their activity will render tumour cells sensitive to virus infection. This differential susceptibility forms the basis for the use of viral-based cancer therapeutics of the present invention for the treatment of tumorous cell growth.

Screening of PKR null mouse strains with several different viruses indicated that PKR null animals are capable of suppressing a number of virus infections including vaccinia, influenza and EMCV. However, Vesicular Stomatitis Virus (VSV) exhibited an ability to infect PKR-/- animals. VSV, a member of the Rhabdovirus family, was observed to kill 100% of PKR null animals following intranasal infection by as little as 50 infectious virus particles (or plaque forming units, pfu). In contrast, over 20,000 times as many VSV particles were required to kill half of infected wild type littermates.

VSV is an enveloped, negative sense RNA virus with a simple five gene genome. This is a very well characterized virus family with several serologically distinct laboratory strains and a multitude of characterized mutants. The natural hosts of VSV include insects, rodents and domestic farm animals. In general, very few North Americans have come in contact with the virus ― most human infections occurring in laboratory personnel and farmers. In humans infections are either asymptomatic or manifested as mild "flu". There are no reported cases of severe illness or death amongst infected humans.

The ability of VSV to selectively infect tumour cells over wild-type cells was also observed. Tumour cell lines, following an overnight infection exhibited a 100 to 1000 times higher rate of infection than that detected in normal primary fibroblasts. Furthermore, the cytopathic effect (cpe) was accelerated in the tumour cell cultures.

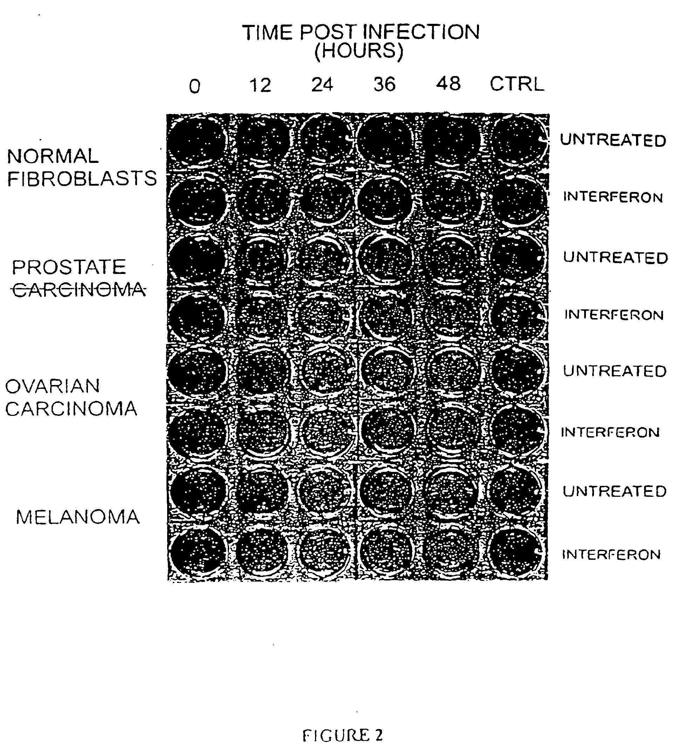

Since PKR is an interferon inducible gene product, pretreatment of cells with interferon prior to exposure to VSV was tested to determined the effect of viral infection. Wild-type cell cultures, that were pretreated with interferon, were resistant to VSV infection, while tumour cell lines, for example, but not limited to, fibrosarcoma, melanoma, prostate carcinoma, leukaemia and ovarian sarcoma, were susceptible to virus infection (see Table 1, Example 2; Figure 2). Lung carcinoma cells (LC80) were also susceptible to VSV infection in the presence and absence of interferon (data not presented). However, several tumour cell lines were resistant to VSV infection in the presence of interferon.

Ovarian carcinoma cells, fibrosarcoma, lung carcinoma, melanoma, prostate carcinoma, lung carcinoma, and leukaemia cells are VSV sensitive, and this sensitivity was maintained in the presence of interferon, therefore, such tumor cells and cancers derived therefrom may be particularly amenable to VSV treatment. However other cancers may also be amenable to viral treatment as described herein. Studies with respect to VSV sensitivity using primary tumour material is readily available in ascites fluid. Further, since the tumour is contained within the peritoneal cavity it may prove particularly suited to localized administration of a virally based therapeutic. In this regard, live tissue from patient's ascitic fluid can be tested for the ability of the tumour cells to support VSV infection in the presence and absence of interferon.

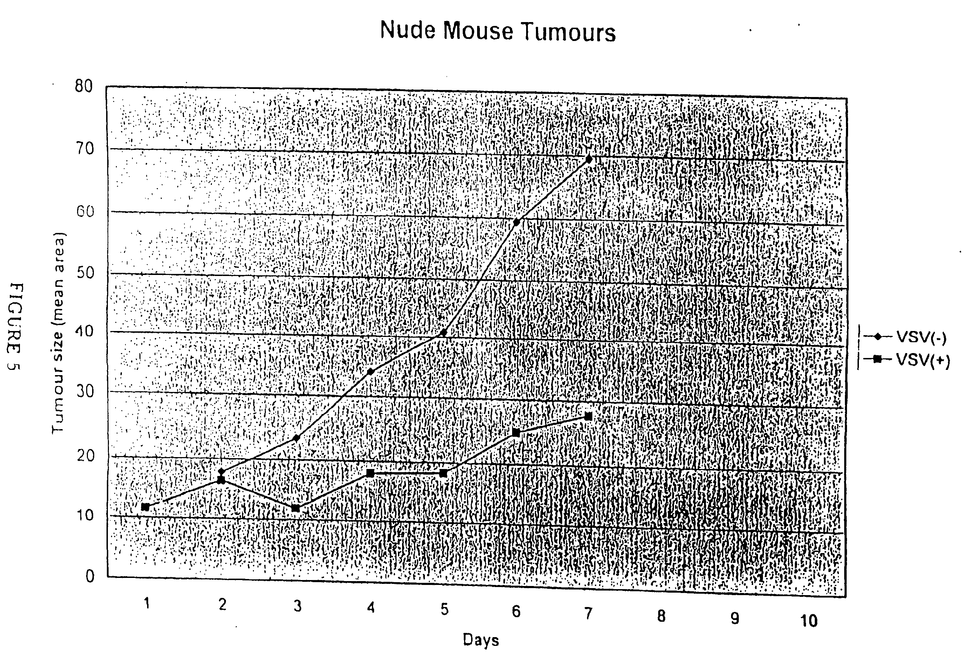

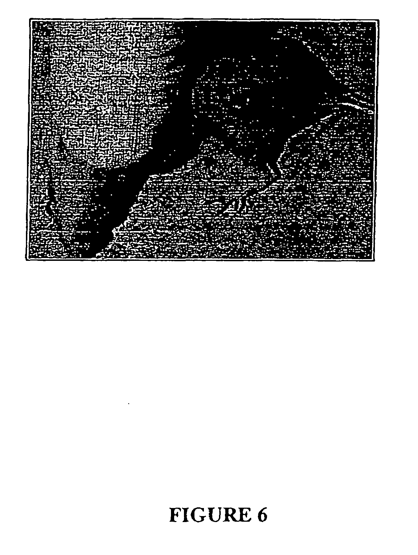

It is expected that VSV will have therapeutic activity *in vivo,* and will have the ability to kill distant (metastatic) tumour growths. To date no significant organ pathology in treated mice have been observed, however, the kinetics of VSV viremia need to be further studied. Nude mice, implanted with human melanoma cells received VSV, or additional melanoma cells infected *in vitro* with VSV (see Example 5), to ensure the continuous production of infective particles to the tumour over a several hour period, via injection (Figure 5). In mock-injected animals (VSV(-); injection with vehicle alone) tumours grew continuously over the course of the experiment. Animals which received only pure virus showed initially continuous growth of tumours over the first four day period, after this time the tumours began to reduce in size and continued to do so over the course of this experiment. Tumours that were injected with infected cells stopped growing and regressed to small hard nodules resembling scar tissue. In some of the larger injected tumours, ulcers formed on the tumour within 1-2 days, (see Figure 6). While both injection of purified virus and infected melanoma cells caused significant regressions, infected producer cells were more effective.

Studies with an immunocompetent mouse tumour model (i.e. as described by Strong et al; 17) will examine the affects of antibody response to therapeutic VSV infection, and determine if VSV infection of tumour cells increases their immunogenicity and promotes recognition of tumour antigens by the host organism.

Primary human bone marrow was also found to be resistant to VSV infection in the absence of interferon pretreatment (see Table 1, Example 2), indicating that these cells have an innate resistance to VSV infection. In contrast two leukemia cell lines (M07E and L1210) were also tested and found to be susceptible to VSV infection as evidenced by cytopathic effect, virus growth and loss of cell viability.

While the results disclosed herein relate to VSV, it is to be understood that one of skill in the art, by following the methods outlined in this document, will be readily able to screen other VSV strains, derivatives of VSV including mutants of VSV, or related viruses for the ability to selectively kill tumour cells. There are several other serologically and biologically distinct strains of VSV, which can be tested for this property. Such VSV strains include, but are not limited to New Jersey, Piry, Coccal, and Chandipura. Identification of other suitable serologically unrelated strains may be useful if sequential VSV injections are required to completely eradicate tumours. Furthermore, combinations of viruses may be used to enhance the cytopathic effect observed with VSV.

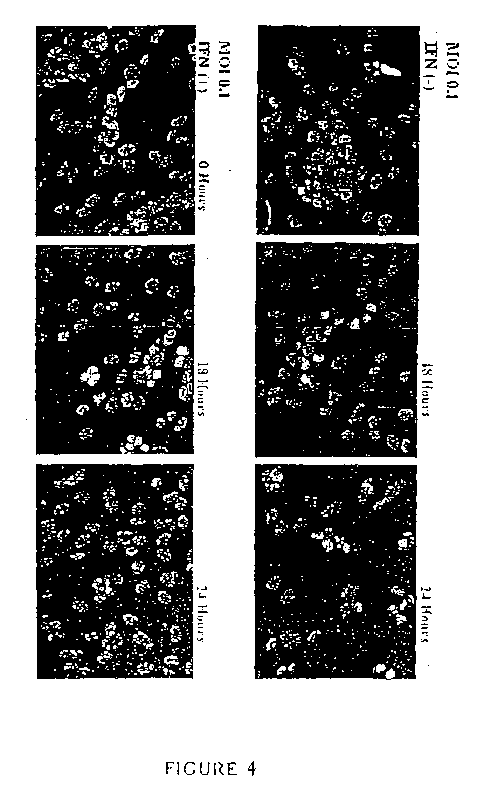

In order to determine whether the presence of either a normal or tumor cell could affect the other cell type (either normal and tumor cell) and alter the resistance or susceptibility of either of these cells to VSV infection, normal cells and fibroblasts were co-cultured in the present of VSV. The culture was infected at an moi of 0.1 pfu/cell and the infection allowed to proceed in the presence or absence of interferon. At 0, 12 and 24 hours (Figure 4) the cultures were fixed and stained with antibodies to large T antigen (red nuclei) to detect the 293T cells and with DAPI (blue nuclei) which stains all cell types (Figure 4). The number of 293T cells (red nuclei) steadily declined during the time course and displayed severely condensed or fragmented nuclei characteristic of a cell dying from virally induced apoptosis. This selective destruction of the transformed cells was seen both in the presence and absence of interferon. The normal fibroblasts did not develop nuclear changes nor were their numbers reduced in response to VSV infection even though 293T cells were producing copious amounts of virus within the co-culture. This indicates that mixtures of cell populations may be treated with VSV while still maintaining tumor cell sensitivity, and normal cell resistance to VSV.

In addition there are a number of mutants of VSV, for example, but not limited to mutants which are impaired in the shut down of host protein synthesis or are more or less sensitive to interferon, which may exhibit differential infection between normal and tumour cells. For example, which is not intended to be limiting in any manner, other viral mutants are known which show tropism for STAT1 or PKR negative cells include an influenza virus strain which is unable to inactivate PKR has been described (36) Adenovirus mutants which lack the PKR inactivating VA gene are known to grow better in the absence of PKR.

As described herein, VSV mutants were isolated that grew poorly on interferon responsive cells. These mutants were selected based upon their ability to form small plaques in monolayers of interferon-responsive cells. On interferon nonresponsive cells (i.e. tumor cells) these mutants form large plaques. The selection of mutants by size of plaque in interferon-responsive cells allows for the isolation of virus that grows poorly in normal cells. However, other VSV mutants may be obtained under different selection criteria. Mutants isolated using interferon-responsive cells were amplified and tested for their ability to kill tumour and normal cells. The rationale here is that VSV mutants, which can induce interferon in target cells, would limit their own replication in an interferon responsive cell population. These same viruses would however have unrestricted growth in tumour cells that lack interferon responsiveness. These mutants are of value, as they have even less cytopathic effect on normal tissues while maintaining oncolytic activity than wild type VSV.

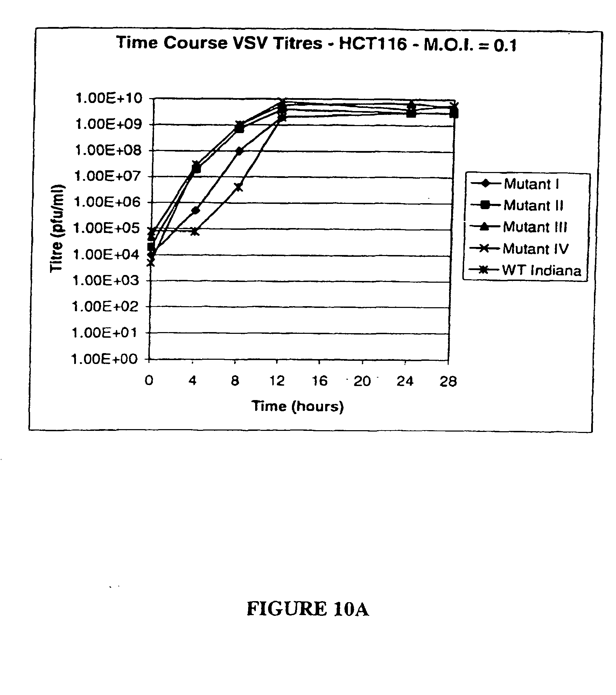

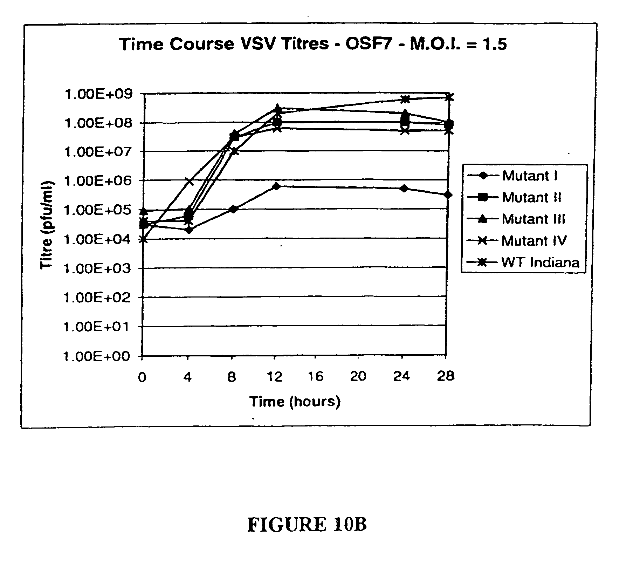

Four mutants (Mut 1-4) were obtained based on their ability to form plaques in monolayers of interferon-responsive cells. These mutants, and wild type virus (moi of 1.0 pfu/cell) were used to infect melanoma cells and normal human foreskin fibroblasts. All of the mutants were able to kill tumour cells efficiently but normal cells infected with the mutants even after long periods of infection appeared completely uninfected. At this same moi wild type VSV demonstrated a cytopathic effect on the normal cells. These results indicate that the mutant virus have a greater therapeutic effect in that they kill tumour cells efficiently while sparing normal cells, and that they also have the ability to produce more virus particles and increase virus spread throughout the tumour (see Example 4). Surprisingly these mutants grew more rapidly than wild type VSV (Indiana) in HCT 116 colon carcinoma cells but not in OSF7 cells (See Example 21 and Figures 10A and 10B). VSV mutants that display rapid growth in the tumour cell of interest but not in normal cells are preferred.

Earlier experiments indicated that PKR -/- mice were killed with VSV by several routes of infection, however, these mice were not affected by intravenous injections of the virus. In order to determine whether plasma components were inactivating the virus upon contact, VSV produced from several sources including within mouse L cells was incubated with human serum (from normal uninfected donor) and the virus titer after incubation determined (Example 6). The viral titer of L cell-produced VSV dropped four hundred fold, while VSV produced in human melanoma cells was unaffected by incubation in plasma. These results indicate that the choice of cell line for the production of VSV is critical. Based on this observation it is possible to screen human cell lines for those that produce optimum amounts of virus that is not sensitive to human serum.

Without wishing to be bound by theory, it may be that the difference in these two virus preparations reflects the nature of the cohort of proteins found on the surface of the virus producing cells. As part of its replicative cycle, VSV buds through the plasma membrane and acquires cellular protein on its envelope. Certain proteins found on L cells when expressed in the contexf of the virus particle could activate complement. Indeed, it has been shown earlier that retrovirus particles produced in certain mouse cells are inactivated by serum while the same virus produced in a subset of human cell lines was unaffected by plasma. (Pensiero, M.N., et al. Hum Gene Ther, 1996. 7:1095-1101).

VSV may be genetically modified in order alter its properties for use *in vivo*. Methods for the genetic modification of VSV are well established within the art. For example a reverse genetic system has been established for VSV (Roberts A. and J.K. Rose. Virology, 1998. 247:1-6) making it possible to alter the genetic properties of the virus. Furthermore, standard techniques well known to one of skill in the art may be used to genetically modify VSV and introduce desired genes within the VSV genome to produce recombinant VSVs (e.g. Sambrook *et al.,* 1989, A Laboratory Manual. New York: Cold Spring Harbor Laboratory Press).

VSV may be targeted to a desired site *in vivo* to increase viral efficacy. For example, modification of VSV G protein to produce fusions that target specific sites may be used to enhance VSV efficiency *in vivo.* However, it is to be understood that other protein targets in addition to the VSV G protein may also be modified to produce such fusion proteins. Such fusion proteins may comprise, for example, but not limited to, Single chain Fv fragments (Lorimer, I.A., et al. Proc. Natl. Acad. Sci. U.S.A., 1996. 93:14815-20) that have specificity for tumour antigens. An example of such a single chain Fv fragment that may be used to prepared a VSV G fusion protein, is an Fv fragment that targets a mutant EGF receptor found on about 80% of human breast tumour cells.

VSV may also be modified to express one or more suicide genes capable of metabolizing a pro-drug into a toxic metabolite thereby permitting VSV infected cells to be killed by administration of a pro-drug. For example, VSV comprising the herpes virus thymidine kinase gene or the cytosine deaminase gene encodes an enzyme that can convert ganciclovir or 5-FC, respectively, into a toxic compound. However, it is to be understood that other suicide genes may also be employed. As it is well established that ganciclovir metabolites kill not only cell expressing HSV TK but also cells in the immediate vicinity, rVSV comprising these suicide genes exhibit several advantages. For example, the effective killing by the virus is increased since one infected cell kills ten or more surrounding tumour cells, furthermore rVSV comprising a suicide gene permits the elimination of virus if desired from an individual infected with the virus. This may be important in situations where it is unclear how VSV may affect an individual. For instance, an immune comprised individual may be unexpectedly susceptible to VSV. Thus the addition of a suicide gene would be an improvement on the safety of the viral therapeutic.

VSV may also be modified by the introduction of a mammalian gene product. Such a mammalian gene product would limit VSV growth in normal cells, but not the growth of VSV in tumour or diseased cells. For example, rVSV capable of expressing one or more transactivators of p53, activates apoptotic pathways in normal cells but not tumor cells. Such rVSVs therefore selectively limit virus spread in normal tissues. However, it is to be understood that other mammalian gene products may also be expressed within VSV for this purpose. Another example, which is not to be considered limiting in any manner is the PKR gene. A rVSV expressing the PKR gene limits virus replication in all normal cells, however, in cells that express PKR inhibitors, the virally encoded PKR is inactivated. An example of a cell that expresses one or more PKR inhibitors is a chronically Hepatitis C infected cell. Since Hepatitis C encodes and expresses two known inhibitors of PKR (i.e. NS5A and E2), a VSV encoded PKR gene product is be neutralized, and VSV allowed to replicate freely.

The above description is not intended to limit the claimed invention in any manner, furthermore, the discussed combination of features might not be absolutely necessary for the inventive solution.

The present invention will be further illustrated in the following examples. However it is to be understood that these examples are for illustrative purposes only, and should not be used to limit the scope of the present invention in any manner.

### Example 1: PKR negative cells are susceptible to VSV infection

### In vivo experiments

Initial studies were directed to identifying viruses that are capable of infecting PKR-/- animals and cells. Using homologous recombination strategies, PKR null mouse strains were generated (35) and tested for their ability to fight virus infections. Since these mice are PKR-/-, they should be susceptible to virus infection. Several species of virus were administered to PKR null animals over a range of of concentrations.

### Infection of PKR null mice:

A PKR null mouse line was generated using conventional knockout technology (Abraham, N., et al., J Biol Chem, 1999. 274:5953-5962.). Groups of five female mice, 3 months of age or greater, were infected intranasally with varying amounts of vesicular stomatitis virus (Indiana strain). Age matched wild type animals were infected in parallel and both sets of animals were monitored on a daily basis for signs of infection. These include, hydration, piloerection, activity level, appetite, hind limb paralysis, respiratory rate, body weight and any other symptoms indicating that the animal was in distress.

Wild type animals showed few and only transient symptoms at multiplicities of infection up to 10⁵ pfu with VSV. In contrast, PKR null animals very rapidly developed dehydration, piloerection, loss of appetite, rapid respiratory rate, decreased activity and squinting crusty eyes. At high doses of VSV infection (10⁵ pfu) the animals showed symptoms in less than 24 hours and usually succumbed to the infection within 48 hours. At doses of infection as low as 25 pfu, 100 percent of the PKR null animals died of VSV infection within 5 days. In separate experiments groups of five wild type and PKR null animals were sacrificed at 48 hours post infection with VSV and organs were removed to assess viral titres. In the PKR animals titres in excess of one million PFU / ml of lung homogenate were found at this time while in wild type animals virus titres ranged from 0 to 100 pfu per ml of lung homogenate. In the wild type and PKR null animals similar amounts of virus were found in the brain. The remainder of the tissues in both mouse strains had undetectable virus at this time post infection.

Vesicular stomatitis virus, a member of the Rhabdovirus family was able to kill 100% of PKR null animals following intranasal infection by as little as 50 infectious virus particles (or plaque forming units, pfu). In contrast, over 20,000 times as many VSV particles were required to kill half of infected normal littermates. These results indicate that PKR null animals are capable of suppressing a number of virus infections including vaccinia, influenza and EMCV. However, VSV exhibited an ability to infect PKR-/- animals. These results also indicate that PKR is required by mammalian cells to resist infections by VSV (Indiana laboratory strain).

### Example 2: Selective killing of tumour cells with VSV

### In vitro experiments

Several tumour cell lines were chosen at random from the Ottawa Regional Cancer Center and tested for their susceptibility to VSV infection. Primary fibroblast cultures from healthy adult volunteers or primary bone marrow samples from healthy donors were used as control cells.

### Infection of tumour cells with VSV:

As a first test of the oncolytic properties of VSV, virus production and cytopathic effect following an overnight incubation with VSV was assessed. Monolayers of cells were incubated with the Indiana strain of VSV at a multiplicity of infection (moi) of 0.1 plaque forming units (pfu). After allowing virus to adsorb for 30 minutes at 37 C, the cultures were rinsed thoroughly with phosphate buffered saline (PBS) and then cultured an additional 18 hours at 37 C. At this time, the cultures were examined microscopically for cytopathic effect (cpe) and photographed. The 18 hour supernatant was removed and virus titres per ml of medium determined. In some experiments, cultures were preincubated for 12 hours with human alpha interferon (100 units/ml) prior to infection.

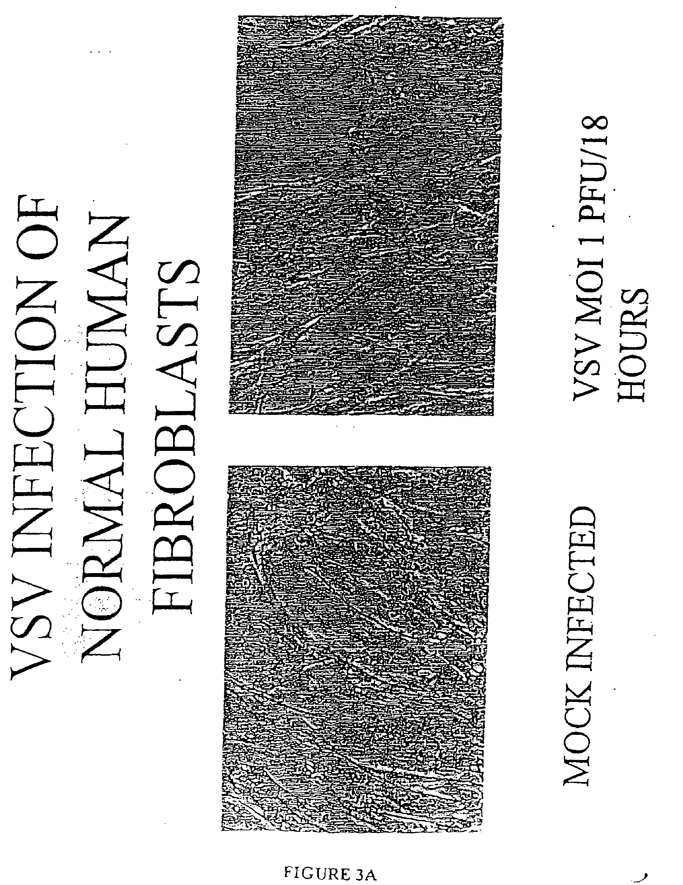

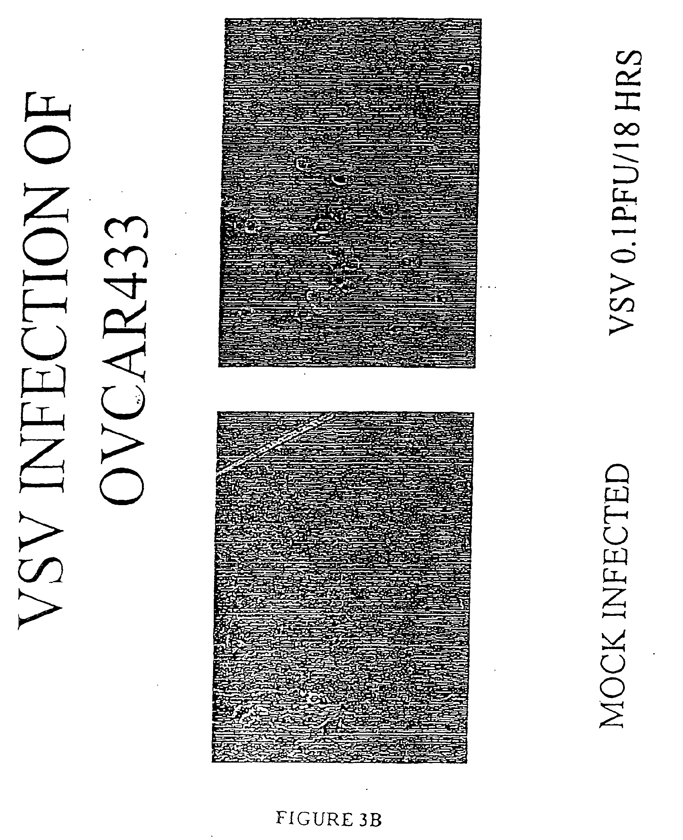

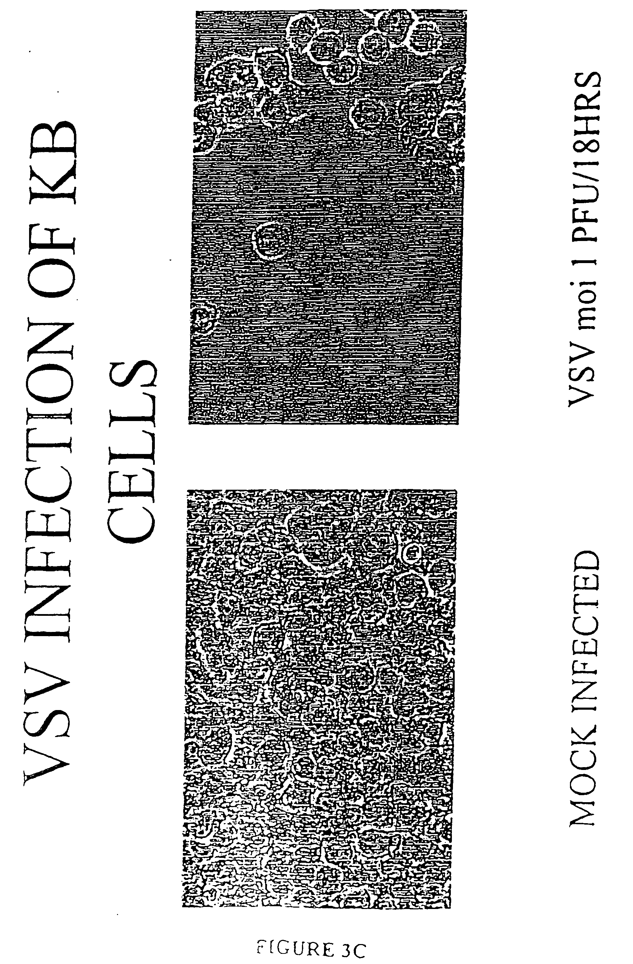

To examine the kinetics of infection of the assorted cell types a modified cpe assay (Heise, C., et al., Nat Med, 1997. 3:639-645) was used. Essentially, monolayers of cells were infected at an moi of 0.1 pfu in a 12 well plate. At time 0 and every 12 hours subsequent up to 48 hours, one well of infected cells was fixed with 0.5 ml Leukostat fixative (Fisher Diagnostics) for 2 minutes. At the end of the experiment monolayers were stained with Leukostat stains 1 and 2 following manufacturers instructions. Since PKR is an interferon inducible gene product, the pretreatment with interferon, 100 units/ml of human alpha interferon 12 hours prior to infection, was tested to determined if interferon could enhance protection within the assorted cell cultures. The data are presented in Table 1 and Figures 2-3.

**TABLE 1: Cell lines tested for VSV sensitivity**

| Cell line | cell type | Reference | Untreated | Interferon |
|---|---|---|---|---|
| | | | Overnight Virus | Overnight |
| | | | Yield | Virus Yield |
| OSF 16 | human normal fibroblast | ORCC ¹ | 1 X 10⁵ pfu | 0 pfu |
| AG1522 | human foreskin fibroblast | [20] | | 0 pfu |
| OSF 7 | human normal fibroblast | ORCC | 1 X 10⁶ | 0 pfu |
| OSF 12 | human normal fibroblast | ORCC | 2 X 10⁵ pfu | 0 pfu |
| MN11 | mouse fibrosarcoma | [21] | 1 X 10⁸ | 1 X 10⁴ |
| A2780 | human ovarian carcinoma | [22] | 2 X 10⁸ | 1 X 10⁷ |
| H-1078 | normal human bone marrow | ORCC | 0 pfu (moi 10 pfu) | Not determined |
| M07E | human leukemic cell line | [23] | 2 X 10⁶ (moi 1.0 pfu) | Not determined |
| L1210 | mouse leukemic cell line | [24] | 4 X 10⁶ | 2 X 10⁴ |
| SK-MEL3 | human melanoma | [25] | Not determined: cpe assay positive | Not determined: cpe assay positive |
| LNCAP | human prostate carcinoma | [26] | Not determined: cpe assay positive | Not determined; cpe assay positive |
| 293T | fibrosarcoma transformed with SV-40 Large T and Adeno E1A | [27] | 1 X 10⁸ | 8 X 10⁷ |
| OVCA 432 | | [28] | 1 X 10⁷ | 0 pfu |
| C13 | ovarian carcinoma | [29] | 1 X 10⁸ | 1 X 10⁵ |
| OVCA 3 | | [30] | 5 X 10⁷ | Not determined |
| COS | Large T transformed simian kidney cell line | [31] | 2 X 10⁸ | Not determined |
| HCT 116 | colon carcinoma | [32] | Not determined: cpe assay positive | Not determined cpe assay positive |
| OVCA 420 | | [28] | 1 X 10⁸ | 3 X 10⁶ |

| | | | | |
|---|---|---|---|---|
| 1 established at the ORCC from forearm biopsy. | | | | |

From the data in Table 1 it can be seen that although normal human fibroblasts can support viral replication, the amount of virus produced and the progression to cell lysis was substantially delayed when compared to tumour cells. An even more substantial difference in virus production was observed following pretreatment with interferon. While normal human fibroblast monolayers were completely protected from the cytolytic affect of VSV by interferon, tumour cells remained sensitive, producing copious amounts of viral particles and rapidly undergoing cytolysis.

Other cells lines, inlcuding a lung carcinoma cell line (LC80) and a leukaemia cell line; AML5 (acute myelogenous leukemia 5) cells were also found to be effectively killed by VSV. In the case of AML5, at a moi of 1.0 pfu/ml cells were completely killed within 24 hours, while at 0.0001 pfu/ml the cells were killed within 72 hours, further indicating the sensitivity of leukaemia cells to VSV.

As can be seen in Figure 2, monolayers of tumour cells were much more rapidly destroyed by VSV infection as compared to normal human fibroblasts. The human melanoma cell line SK-MEL3, the LNCaP prostate cancer cell line and the ovarian carcinoma cell A2780 all showed substantial cpe as early as 12 hours post infection. Although the normal human fibroblast cultures were infected and capable of producing virus (see Table 1), the kinetics of infection was substantially slower than in the three tumour cell lines tested in this experiment. In addition, as with the overnight virus growth assay (Table 1, Figure 2), interferon alpha treatment completely protected the normal human fibroblasts, but was ineffective at protecting the three tumor cell lines from the cytopathic effect of VSV.

The results obtained for Table 1 demonstrate that a screening strategy for determuning the types of tumours which are susceptible to killing by VSV may be employed using for example, but not limited to, the NIH/NCI standard panel of tumour cell lines available from ATCC. These cell lines are screened in order to determine the time to complete cpe and/or virus growth using various initial multiplicities of infection. These experiments are done in the presence and absence of interferon so that the number of and types of tumours that are VSV sensitive and are resistant to interferon's antiviral activity are determined.

### VSV Treatment of Leukemia

VSV does not productively infect bone marrow stem cells, even at high moi of 10 pfu/cell (H-1078; Table 1). The treated cultures retained all of their stem cell characteristics. Two leukemia cell lines (MO7E and L1210; Table 1) were killed following an overnight infection and produced large amounts of virus.

To determine whether VSV could kill primary leukemia cells from a cancer patient, a peripheral blood sample was obtained from an AML patient and white blood cells collected and plated in RPMI media plus 10% FBS (10⁷/well in 6 well plate, each infection in duplicate). Cells were mock infected or infected at an moi of 10.0/cell. VSV selectively killed myeloid leukemic cells as indicated by the decrease in the percentage of blast cells (leukemic blasts), while the overall cell number was minimally affected (i.e. neutrophils flourished). The leukemic sample produced titres of VSV exceeding 10⁷ pfu/ml at 16 hours post infection. The number of blast cells in the sample was dramatically reduced at 21 hours post infection while the proportion of normal neutrophils increased. Mock infected cells (-VSV) contained almost 70% blast cells in a monolayer, while in cells infected with VSV (+VSV) normal cells predominated. These results demonstrate VSV is able to preferentially kill primary leukemic blast cells while sparing normal blood cells.

### Example 3: Killing of tumour cells in mixed cultures:

Normal human fibroblasts and 293T tumour cells were co-cultured in a 50:50 mixture. Since 293T cells express the large T antigen which is not found in normal cells, the two cell types can be distinguished by immunofluoresence.

In this experiment cultures were infected at an moi of 0.1 pfu/cell and the infection allowed to proceed in the presence or absence of interferon. At 0, 18 and 24 hours (Figure 4) the cultures were fixed and stained with antibodies to large T antigen (red nuclei) to detect the 293T cells and with DAPI (blue nuclei) which stains all cell types (Figure 4). Initially both cell types displayed a spindle-like morphology with large oval nuclei. After 18 hours the number of 293T cells (red nuclei) were reduced and many of the remaining 293T cells displayed altered nuclear morphology. By 24 hours post-infection very few 293T cells were detected and those few that remained displayed severely condensed or fragmented nuclei characteristic of a cell dying from virally induced apoptosis.

This selective destruction of the transformed cells was seen both in the presence and absence of interferon. The normal fibroblasts did not develop nuclear changes nor were their numbers reduced in response to VSV infection even though 293T cells were producing copious amounts of virus within the co-culture.

### Example 4: VSV Mutants as Oncolytic Agents:

VSV mutants were isolated based upon their ability to form small plaques in monolayers of interferon-responsive cells, as compared to the size of plaques in monolayers of interferon-nonresponsive cells. Viral isolates, which form small plaques in interferon-responsive cells were picked, amplified and re-cloned. Mutants isolated in this way were amplified and tested for their ability to kill tumour and normal cells. The rationale here is that VSV mutants, which can induce interferon in target cells, would limit their own replication in an interferon responsive cell population. These same viruses would however have unrestricted growth in tumour cells that lack interferon responsiveness. These mutants would be of value, as they should have even less cytopathic effect on normal tissues while maintaining oncolytic activity.

Four mutants (Mut 1-4) were obtained based on their ability to form small plaques in monolayers of interferon-responsive cells. These mutants were initially identified by Dr. Lauren Poliquin (University of Quebec at Montreal) and provided by him. After five rounds of plaque purification, these mutants and wild type virus (moi of 1.0 pfu/cell) were used to infect melanoma cells and normal human foreskin fibroblasts and titres of released virus determined 12 and 24 hours post infection.

All of the mutants were able to kill tumour cells efficiently but normal cells infected with the mutants even after long time points appeared completely uninfected. At this same moi wild type VSV demonstrated a cytopathic effect on the normal cells. It was also observed that all of the VSV mutants produced approximately ten times more virus than the wild type VSV following an overnight infection of melanoma cells. On normal cells, while the Mutants 1-4 had significantly less cytopathic effect than wild type VSV, similar amounts of virus were produced from the infected cultures. These results indicate that the mutant virus have a greater therapeutic effect in that they kill tumour cells efficiently while sparing normal cells, and that they also have the ability to produce more virus particles and increase virus spread throughout the tumour.

### Example 5: Infection of Nude Mice Bearing Human Tumour Xenografts:

Nude mice were implanted with human melanoma cells and divided into groups. One group received a mock injection (VSV(-)), and the other were injected with wild type VSV or injected with additional melanoma cells infected *in vitro* with VSV for one hour prior to injection into the tumour site in order to deliver cells that would continuously produce infective particles to the tumour over a several hour period (VSV(+)). The results of these experiments are seen in Figure 5 which shows the average of the tumour area with time in treated and mock injected animals.

In the case of mock-injected animals (VSV(-); injection with vehicle alone) tumours grew continuously over the course of the experiment. Animals which received only pure virus showed initially continuous growth of tumours although at day 4 post infection the tumours began to shrink and continued to do so over the course of this experiment. Tumours that were injected with infected cells demonstrated the most dramatic regressions. Essentially most tumours stopped growing and regressed to small hard nodules resembling scar tissue.

In some of the larger injected tumours, ulcers formed on the tumour within 1-2 days, (see Figure 6), followed by continuous shrinkage of the once rapidly growing malignancy. While both injection of purified virus and infected melanoma cells caused significant regressions, infected producer cells were more effective.

### Example 6: The choice of cell line for producing VSV affects sensitivity of the virus to plasma:

Earlier experiments indicated that PKR -/- mice were killed with VSV by several routes of infection, however, these mice were not affected by intravenous injections of the virus. Without wishing to be bound by theory, this could be because the PKR -/- vascular endothelial cells provide a barrier to tissue infection or because plasma components were inactivating the virus upon contact. To test this latter idea VSV produced from several sources including within mouse L cells was incubated with human serum (from normal uninfected donor) and the virus titer after incubation determined.

Following incubation of VSV in human serum, the viral titer of L cell-produced VSV dropped four hundred fold. On the on the other hand VSV produced in human melanoma cells was unaffected by incubation in plasma.

These results indicate that the choice of cell line for the production of VSV is critical. Based on this observation it is possible to screen human cell lines for those that produce optimum amounts of virus that is not sensitive to human serum.

### Example 7: Use of VSV to treat chronic infections

Some human disorders arise as a result of chronic viral infections including latent herpes infection, hepatitis, AIDS and cervical cancer. In each of these cases, the causative viral agent has evolved mechanisms to inactivate components of the interferon response pathway including PKR (e.g. Chelbi-Alix, M.K. and H. de The, Oncogene, 1999. 18.935-941;. Gale, M.J., Jr., et al., Virology, t997. 230: 217-227; Gale, M.J., et al., Clin Diagn Virol, 1998. 10:157-162; Gale, M., Jr. and M.G. Katze, Methods, 1997. 11:383-401; Barnard, P. and N.A. McMillan, Virology, 1999. 259:305-313). Therefore, the administration of VSV, or interferon inducing VSV mutants, or a combination thereof, to individuals suffering from these disorders, selectively ablates the chronically infected cells. Further therapeutic efficacy could be found by targeting through cell or viral receptors only the chronically infected cells.

### Example 8: Genetic modification of VSV

A reverse genetic system has been established for VSV (Roberts A. and J.K. Rose, Virology. 1998. 247:1-6) making it possible to alter the genetic properties of the virus.

### Targeting VSV to desired sites in vivo

Presently VSV can bind to most mammalian cell types although its replication once inside the cell can be restricted (i.e. by interferon responsive gene products including PKR). Thus the effective dose of virus that can actually find target cells (i.e. tumour cells) for productive infection can be greatly limited simply by the "sink" that other normal tissues provide. Therefore, VSV may be genetically modified in order to bind and infect only tumour cells.

Recombinant DNA techniques well known in the art (e.g. Sambrook *et al*., 1989, A Laboratory Manual. New York: Cold Spring Harbor Laboratory Press) are used to modify VSV G protein. Single chain Fv fragrnents (Lorimer, I.A., et al. Proc. Natl. Acad. Sci. U.S.A., 1996. 93:14815-20) that have specificity for tumour antigens are fused to VSV G protein. An example of such a single chain Fv fragment is one that targets the mutant EGF receptor that is found on about 80% of human breast tumour cells.

### Expression of suicide genes within VSV

The VSV genome is modified so that it comprises the herpes virus thymidine kinase gene or the cytosine deaminase gene. Both of these genes encode enzymes which can convert pro-drugs into toxic compounds (e.g. ganciclovir or 5-FC). Viruses modified in this way express these suicide genes, thereby permitting VSV infected cells to be killed by administration of the pro-drug. This provides two advantages since(1) it is well established that ganciclovir metabolites kill not only cell expressing HSV TK but also can cells in the immediate vicinity. This "by-stander effect" can increase the effective killing by the virus (i.e. one infected cell could result in the killing of ten or more surrounding tumour cells); and (2) having a VSV with a suicide gene could allow the elimination of virus if desired from an individual infected with the virus.

### Controlling VSV growth in vivo

A mammalian gene product is introduced within VSV to limit VSV growth in normal cells, but this gene product does not affect VSV growth in tumour or diseased cells.

Recombinant VSVs (rVSV) comprising one or more transactivators of p53, activate apoptotic pathways in normal cells but not tumour cells. Such rVSVs limit virus spread in normal tissues but allow virus growth in tumour cells.

rVSV comprising the PKR gene limits virus replication in all normal cells, however, in cells that express PKR inhibitors, the virally encoded PKR is inactivated. An example of a cell that expresses one or more PKR inhibitors is a chronically Hepatitis C infected cell. Since Hepatitis C encodes and expresses two known inhibitors of PKR (i.e. NS5A and E2), a VSV encoded PKR gene product is be neutralized, and VSV allowed to replicate freely

### Example 9. Progressive loss of interferon responsiveness with oncogenic transformation

Murine fibroblasts at various stages of transformation, either pretreated with 100 units of interferon alpha or left untreated, were infected with WT Indiana VSV at an MOI of 0.1 pfu/cell. Viral production was measured 18 hours pi by standard plaque assay. MEF: mouse fibroblast primary cultures isolated from Balb/C mouse embryos. NIH 3T3 cells: immortalized mouse embryo fibroblasts. PVSrc: NIH 3T3 cells transformed with the viral src gene. MOP 8: NIH 3T3 cells transformed with the polyoma virus Large T antigen. Results are shown in Table 2.

In this example, loss of interferon responsiveness correlates with susceptibility to VSV infection and progression of the malignant phenotype. The MEF cells are mortal (ie have a limited lifespan in culture) and completely interferon responsive. NIH 3T3 cells although not tumourigenic are immortalized and are about ten thousand fold less responsive to interferon than MEFs. The PVSrc and MOP 8 cells are fully tumourigenic, support robust VSV replication and are minimally protected by interferon treatment.

**TABLE 2**

| **Cell Line** | **Viral Titre (pfu/ml)** | |
|---|---|---|
| | **Untreated** | **IFN-**α |
| | | |
| MEF (Mouse Embryonic Fibroblast) | 4 x 10⁶ | <10 |
| NIH3T3 | 8 x 10⁷ | 1 x 10⁴ |
| PVSrc | 3 x 10⁹ | 2 x 10⁷ |
| MOP 8 | 1 x 10⁸ | 5 x 10⁶ |

### Example 10. Virus yield after overnight infection of various cell lines either untreated or treated with IFN

A variety of normal and transformed cell lines were either untreated or pretreated with 100 units of IFN-α, infected at an MOI of 0.1 pfu/ml with WT Indiana VSV and incubated for 18 hours at 37 °C. Culture media from each sample was titred for VSV production. Results are shown in Table 3.

This example demonstrates that viral production is ten to ten thousand times more efficient in a range of tumour cell types as compared to normal primary tissues. In the presence of interferon alpha, virus production in normal primary cells is almost completely blocked while in tumour cells interferon has little or no effect on VSV replication.

**TABLE 3**

| **Cell Line** | **Viral Titre (pfu/ml)** | |
|---|---|---|
| | **Untreated** | **IFN-**α |
| OSF7 (primary normal human fibroblast) | 1 x10⁶ | <10 |
| OSF12 (primary normal human fibroblast) | 2 x 10⁵ | <10 |
| OSF16 (primary normal human fibroblast) | 1 x10⁵ | <10 |
| PrEC (primary normal human prostate epithelium) | 8 x 10⁶ | <10 |
| HOSE (primary normal human ovarian surface epithelium) | 1 x10⁷ | <1000 |
| A2780 (human ovarian carcinoma) | 2 x10⁸ | 1x10⁷ |
| OVCA 420 (human ovarian carcinoma) | 1 x 10⁸ | 3 x 10⁶ |
| C13 (human ovarian carcinoma) | 1 x10⁸ | 1 x 10⁵ |
| LC80 (human lung carcinoma) | 2 x 10⁹ | 6 x10⁷ |
| SK-MEL3 (human melanoma) | 1 x10⁹ | 1 x 10⁹ |
| LNCAP (human prostate carcinoma) | 4 x 10⁹ | 5 x 10⁹ |
| HCT116 (human colon carcinoma) | 1 x 10⁹ | 2 x 10⁹ |
| 293T (HEK cells transformed with T antigen and Ad virus ElA) | 1 x10⁸ | 8 x10⁷ |

### Example 11. LD₅₀ for WT and mutant VSV delivered intranasally to KR^{+/-}(129xBalb/c) mice.

8-10 week old female mice were anaesthetised and infected intranasally with virus diluted in 50 µl of phosphate buffered saline (PBS) into the nares of each animal (PKR^{+/-} ; 129 x Balb/c strain). Lethal dose 50 values were calculated using the Korler-Spearman method. Results are shown in Table 4.

This example demonstrates that mutants I, II and III in particular, are attenuated as compared to the wild type Indiana strain of virus when tested for toxicity in 129 X Balb/c mice.

**TABLE 4**

| **Virus** | **Intranasal LD**_{**50**} **(pfu)** |
|---|---|
| WT Indiana | 1 x 10⁴ |
| Mutant I | 1 x 10¹⁰ |
| Mutant II | >1 x 10¹⁰ |
| Mutant III | 3 x 10⁸ |
| Mutant IV | <1 x10⁵ |

### Example 12. PKR^{-/-} mice are exquisitely sensitive to VSV compared to various PKR^{+/+} mouse strains.

PKR^{-/-} and PKR^{+/+} mice were infected intranasally at various doses and their survival monitored over time. All PKR^{-/-} mice succumbed to the infection between days 2 and 5 depending on the dose, while control mice remained alive beyond this point. Results are shown in Table 5.

This example demonstrates the importance of the PKR gene product in the resistance of mice to VSV infection.

**TABLE 5**

| | **Genetic Background** | **IN Dose (pfu)** | **Survival at day 5** |
|---|---|---|---|
| PKR^{+/+} | Balb/c | 5x10⁴ | 5/5 |
| | CD-1 | 5x10⁴ | 5/5 |
| | Balb/c x 129 | 5x10⁴ | 5/5 |
| | | | |
| PKR^{-/-} | Balb/c x 129 | 5x10⁴ | 0/5 |
| | | 5x10³ | 0/4 |
| | | 5x10² | 0/3 |
| | | 5x10¹ | 0/3 |

### Example 13. AML3 cells die by apopotosis following infection with VSV

OCI/AML3 (acute myelogenous leukemia) cells were infected with VSV at an MOI of 3.0 pfu/cell. Fourteen and twenty hours post-infection unfixed samples were analyzed. Apoptotic cells with phosphatidylserine membrane translocation were detected by flow cytometry using Annexin-V-Biotin-X/NeutrAvidin-PE red fluorescent protein (Molecular Probes). Mitochondrial membrane depolarization in early apoptotic cells were analyzed by flow cytometry using JC-1 potential-sensitive dye (Molecular Probes). JC-1 is accumulated by polarized mitochondria shifting fluorescence emission from green to red spectra. Non-viable AML3 cells were identified using Ethidium (EthD-1) homodimer-1 red fluorescent vital dye (Molecular probes). Assays were performed following the manufacturers specifieatians. Results are shown in Table 6.

This example demonstrates that VSV kills AML cells at least in part through a virally induced apoptotic pathway.

**TABLE6**

| | **Tests** | | **MOI 0.0** | **MOI 3.0** | **Net Positive (Dead)** |
|---|---|---|---|---|---|
| | | | **Percent Positive** | **Percent Positive** | |
| **14 hrs p.i.** | EthD-1 | | 6.6 | 32.3 | 25.7 |
| | Annexin V | | 14.3 | 52.7 | 38.4 |
| | JC-1 | | 7.5 | 21.4 | 13.4 |
| | | | | | |
| | | | | | |
| | | | | | |
| **20 hrs p.i.** | EthD-I positive | | 6.4 | 58.5 | 52.1 |
| | Annexin-V positive | | 10.8 | 79.6 | 68.8 |
| | IC-1 | | 3.9 | 43.2 | 39.3 |

### Example 14: Mutant VSV strains infect and kill AML cells

OCI/AML3 (acute myelogenous leukemia) cells were infected at an MOI of 1.0 and incubated for 23 hours. Unfixed cells were stained with Eth-D1 (ethidium dimer, Molecular Probes) to detect non-viable cells following manufacturers specifications. Number of stained cells per 10.000 counted used to calculate percent dead. Results are shown in Table 7.

This example demonstrates that the mutant VSV strains used are as effective as the wild type Indiana strain in killing AML cells.

**TABLE 7**

| | | | | | | | |
|---|---|---|---|---|---|---|---|
| | Mock | WT IND | Mut I | Mut D | Mut III | Mut IV | Mut V |
| Percent Dead | 30.0 | 64.7 | 60.7 | 86.7 | 72.1 | 74.4 | 82.8 |

### Example 15. VSV and VSV infected cells exhibit antitumor activity against human melanoma xenografts in nude mice.

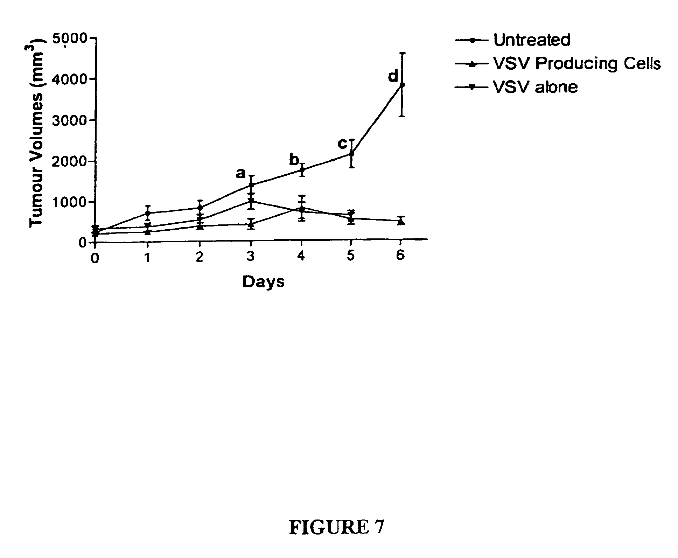

SK-MEL 3 (melanoma) derived tumours were developed in 8-10 week old female Balb/c athymic mice. On day 0, tumours were either left untreated or were infected with 10⁸ pfu WT Indiana VSV in culture media or 2.5x 10⁶ WT Indiana VSV infected SK-MEL 3 cells (VSV producing cells). Statistical differences were calculated between treated and untreated groups at each data point with the following confidence values (b: p<0.01; c: p<0.001; d: p=0.007). Results are shown in Figure 7. On day 3, only tumours treated with VSV producing cells were significantly smaller than untreated tumours (a: p<0.001). No statistically significant differences in tumour volumes between groups were apparent from day 0 to day 2. Data points represent means +/- SEM from multiple tumours (untreated n=8; VSV producing cells n=8; VSV alone n=4).

This example demonstrates that a single injection of VSV. directly into solid tumours profoundly affects tumour growth resulting in partial to complete regression. The use of infected tumour cells as a vehicle to deliver virus is also efficacious.

### Example 16 : PKR7 mice are acutely sensitive to intranasal VSV infection and demonstrate a deficiency in IFN mediated resistance.

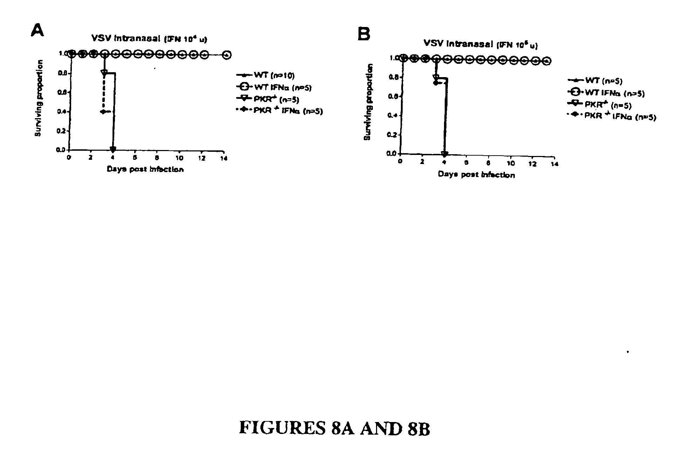

(A & B) PKR^{-/-} and control mice (Balb/c X 129) were infected intranasally with 5x10⁴ pfu of VSV and monitored for morbidity and survival over the course of 14 days. after which remaining animals were deemed to have survived the infection. Results are shown in Figures 8A and 8B. PKR^{-/-} mice showed a severe decrease in survival compared to control mice (WT), succumbing by day 3 or 4, while all control mice survived the infection. IFN-α/β pretreatment (18 h prior to infection) with either 2 x10⁴ IU (Fig. 8A) or 2 x 10⁵ IU (Fig. 8B) had no protective effect in PKR^{-/-} animals.

This example demonstrates that a single defect in the interferon pathway (absence of PKR gene product) is sufficient to render mice unable to resist VSV infections. This defect cannot be rescued by interferon.

### Example 17. Interferon can protect xenograft bearing nude mice during VSV treatment

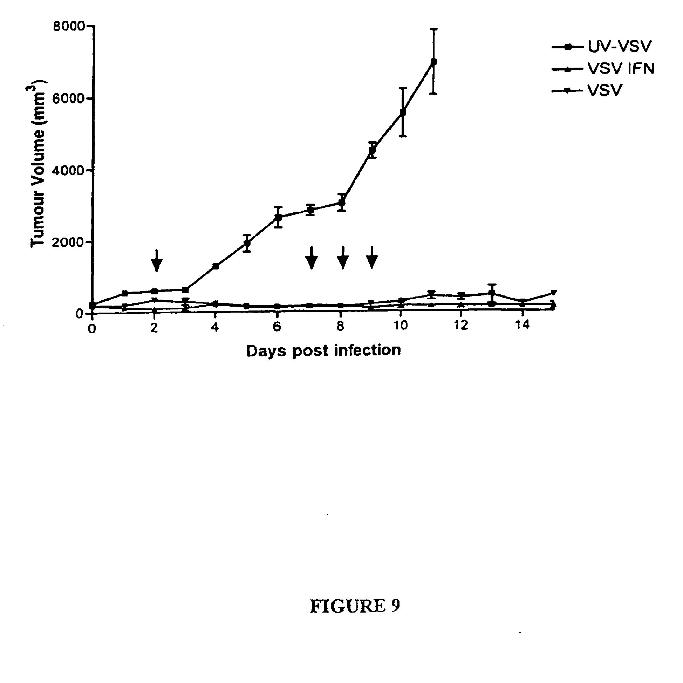

SK-MEL 3 melanoma cells were injected intradermally into CD-1 athymic nude mice. On day 0, tumours were injected with either live WT Indiana VSV (1 x 10⁹ pfu) or an equivalent amount of UV inactivated VSV, and measured daily. Results are shown in Figure 9. Interferon was administered to a subset of animals (VSV IFN) at the times indicated (black arrows). (UV-VSV n=4; VSV IFN n=6; VSV n=6). In these experiments a single intratumoural injection of VSV is tumour-inhibiting in all cases. All tumours had at least a partial regression and in three of twelve mice treated a complete tumour regression. Tumours receiving UV inactivated virus continued to grow unabated until these animals were sacrificed at day 11. Nude mice not receiving interferon and injected with live virus began to die at day 10 and only two of six remained viable by day 15. In contrast, all interferon treated, infected, nude mice were protected from VSV toxicity and remained symptom free for more than 45 days.

This example demonstrates that a single intratumoural injection of live VSV is efficacious against tumours. Furthermore infected, tumour bearing, nude mice can be rescued from VSV toxicity by interferon injection.

### Example 18. VSV infects and kills leukemia and myeloma cells.

The indicated cell lines were infected with VSV Indiana HR strain at a multiplicity of infection of one plaque forming unit per cell. At 24, 48 and 72 hours post infection (p.i.) samples were taken from the infected cultures and stained directly with propidium iodide following manufacturers instructions (Molecular Probes). Samples were then analysed by flow cytometry using the FACSsort WinMDI Version 2.7 program. In Table 8 the percentage of cells dead for each leukemic cell type is shown for the indicated times post infection.

This example shows that VSV is able to infect and kill a diverse set of leukemia types. The K-562 cell is isolated from a chronic myelogenous leukemia (CML) patient while MOLT-4 is a T cell leukemia and SR and H929 are myelomas.

**TABLE 8**

| *Cell Line* | 24 hr. p.i. | 48 hr. p.i. | 72 hr. p.i. |
|---|---|---|---|
| K-562 (CML) | 15.38% | 52.36% | N/D |
| MOLT-4 (T cell Leukemia) | 53.94 % | 48.80 % | N/D |
| SR (Myeloma) | 32.10 % | 46.38 % | N/D |
| H929(Myeloma) | 10.73 % | 17.35 % | 64.41 % |

### Example 19: Vesicular stomatitis virus (VSV) strains including wild type Indiana and five attenuated VSV mutants demonstrate selective cytotoxicity toward human prostate carcinoma cells compared to normal human fibroblasts.

Vesicular stomatitis virus strains including wild type Indiana and attenuated mutant strains I (TR 1026). II (TR1026R). III (TP3), IV (TP6) and V (G31) were obtained from Dr. Lauren Poliquin, University of Quebec at Montreal. Each of these virus strains was plaque purified five times prior to use in this experiment.

Human prostate carcinoma cells (LNCAP) and normal human cells (OSF 7 forearm fibroblast) were grown in 96-well tissue culture plates to a density of approximately 5 x 10⁴ cells per well. Virus was added in 10-fold dilutions ranging from 5 x 10⁵ pfu to 5 pfu. Control wells with no virus were included on each plate. The plates were incubated for 48 hours at 37 °C in 5% CO2. Cytotoxicity was quantified using a colorimetric MTS ((3-[4,5-dimethylthiazol-2-yl]-5-[3-carboxymethoxyphenyl]-2-[4-sulfophenyl]-2H-tetrazolium, inner salt) assay (CellTiter 96 Aqueous, catalog #G1112, Promega Corporation, Madison WI 53711-5399), monitored at 490 nm, that detects mitochondrial enzyme activity. The amount of cell killing in the virus treated wells was determined by the loss in viability in the virus treated wells relative to the untreated wells. The data was plotted graphically as pfu/cell vs. percentage cell killing relative to control. The TC50 for these cells was calculated as the amount of virus in pfu/cell causing a 50% reduction in the amount of viable cells. Lower TC50 values reflect increased sensitivity of the cells to the lytic effects of the virus. The *in vitro* therapeutic index for each VSV strain was calculated as the ratio of TC50 for the OSF7 cells compared to the TC50 for the LNCAP cells.

The results are shown in Table 9. Wild type VSV-Indiana and each of the five mutants demonstrated a high degree of cytotoxicity toward the human prostate carcinoma cells as reflected in the low TC50 values, all less than 0.01 pfu/cell. The normal human fibroblasts cells were one to more than 3 orders of magnitude more resistant to the cytotoxic effects of all six VSV strains. All five mutants had less toxicity on the normal OSF7 fibroblasts cells and had a higher *in vitro* therapeutic index than the wild-type Indiana VSV.

**TABLE 9. Cytotoxicity Assay Results for VSV Strains (Wild type Indiana and Mutants I through V) Against Prostate Carcinoma Cells and Normal Fibroblasts.**

| | | | | | | |
|---|---|---|---|---|---|---|
| | Mutant | Mutant | Mutant | Mutant | Mutant | WT |
| | I | II | III | IV | V | Indiana |
| | | | | | | |
| LNCAP Prostate | 0.0064 | 0.0048 | 0.0014 | 0.0006 | 0.0012 | 0.0017 |
| Carcinoma TC50 (pfu/cell) | | | | | | |
| OSF7 Normal Fibroblasts | >42 | 22 | 4.3 | 0.031 | 9.8 | 0.022 |
| TC50 (pfu/cell) | | | | | | |
| Therapeutic Index | >6562 | 4583 | 3071 | 52 | 8167 | 13 |
| (TC50 OSF7/ TC50 LNCAPP) | | | | | | |

### Example 20: Virus Production from Tumour Cells and Normal Cells Infected with Wild Type Indiana and Various Mutant VSV Strains

HCT 116 colon carcinoma cells and OSF 7 forearm fibroblasts were grown to confluence in 35 mm tissue culture dishes. Media was removed and virus was added in a volume of 30 µl with a multiplicity of infection of 0.1 pfu/cell for the HCT 116 cells and 1.5 pfu/cell for the OSF 7 cells. After a 1 hour incubation period at 37°C, 5% C02, 1 ml of tissue culture media was added to the dishes. Results are shown in Figures 10A and B. At the indicated time points, 10 µl samples of media were removed from the dishes. The virus titre of these samples was determined by a plaque assay.

This example demonstrates the rapid replication kinetics of wild type and mutant VSV strains in HCT 116 colon carcinoma cells. All four mutant VSV strains had more rapid growth in HCT116 tumor cells than the wild type VSV. Note that in the normal OSF-7 cell cultures a ten fold higher input of virus is required to attain similar replication kinetics.

### Example 21. Malignant cells are rapidly killed following VSV (WT Indiana) infection and are not protected by IFN-α.

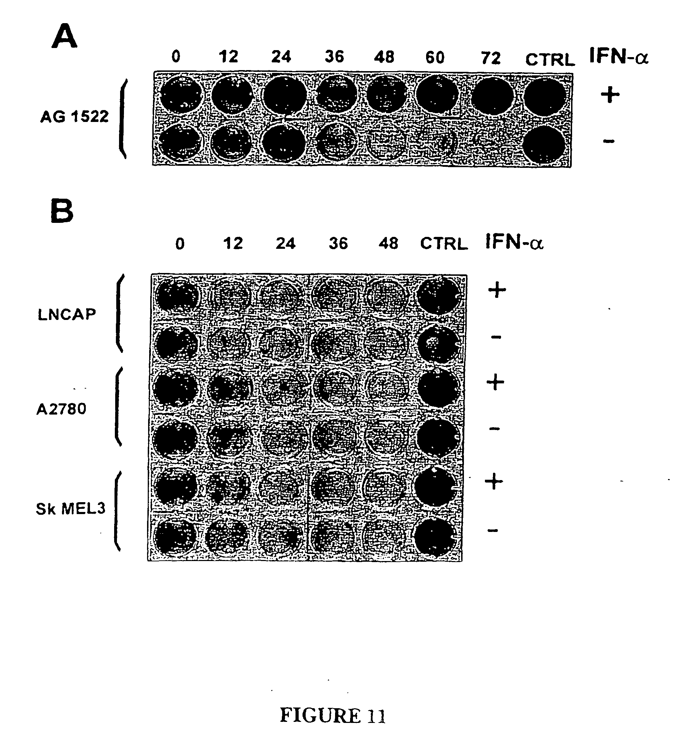

Monolayers of normal primary human fibroblasts (AG 1522) and several tumour cell lines were either untreated or pretreated with IFN-α(100 units) and then infected with VSV at an MOI of 0. 1 pfu/ml. At 12 hours increments the infections were terminated by cell fixation and staining to determine the kinetics of cell killing. Control (CNTL) monolayers were left to grow, uninfected, over the course of the experiment and therefore stain more intensely. Results are shown in Figure 11. LNCAP is a human prostate carcinoma; A2780 is an human ovarian epithelial carcinoma, and Sk MEL3 is a human melanoma.

This example demonstrates the rapid kinetics of tumour cell killing by VSV Indiana even in the presence of interferon alpha. While normal cells are also killed by VSV, the kinetics are slower and normal cells can be completely protected by interferon alpha.

### Example 22: VSV induced cytopathic effect visible in human melanoma cells but not in primary human cells with or without IFN-α.

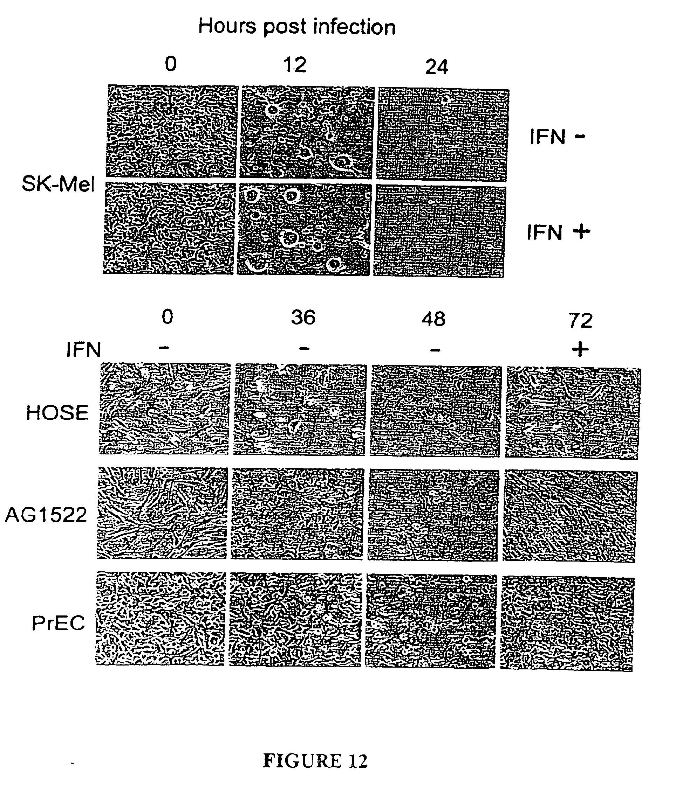

Gelatin-coated coverslips with normal human cells and SK-MEL3 cells untreated or pretreated with IFN-α (100 U/ml) were infected with WT Indiana VSV at an MOI of 0.1 pfu/ml. Results are shown in Figure 12. The human melanoma cells (SK-MEL3) displayed cpe at 12 hours post-infection even in the presence of interferon. At 24 hours post-infection these malignant cells had died and lifted from the coverslip. Human primary cells including foreskin fibroblasts (AG1522), ovarian surface epithelial cells (HOSE) and prostate epithelial cells (PrEC) did not show CPE (cytopathic effect) until 36 hours in the absence of interferon and were completely protected in the presence of interferon beyond 72 hours post-infection.

This example demonstrates that VSV Indiana is able to rapidly destroy melanoma cells even in the presence of interferon alpha whereas normal fibroblasts and epithelial cells are slower to be killed and can be completely protected by interferon alpha.

### Example 23. VSV selectively kills transformed cells co-cultured with normal fibroblasts.

Equal numbers of 293T cells (human embryo kidney cells transformed with adenovirus EIA and Large T antigen) and normal human foreskin fibroblasts were plated on gelatin-coated coverslips and infected (WT Indiana VSV) at an MOI of 0.1 both in the presence and absence of interferon. Cells were fixed at 12 (not shown), 24 and 36 hours post-infection. Fixed cells were stained with an anti-TAg antibody and DAPL The red staining 293T cells were quickly killed as early as 12 hours post-infection, regardless of interferon treatment, with those few remaining cells displaying condensed or fragmented nuclei. The normal fibroblasts displayed altered nuclei by 36 hours post-infection in the absence of interferon but were protected from the virus in the presence of interferon beyond this time point.

This example demonstrates that in a mixed culture of normal and tumour cells. VSV Indiana preferentially replicates and kills tumour cells. Normal cells in the infected co-cultures are slower to die and can be completely rescued by interferon treatment.

### Example 24. Efficacy of a single intravenous dose of mutant VSV in treating human melanoma xenografts in nude mice.

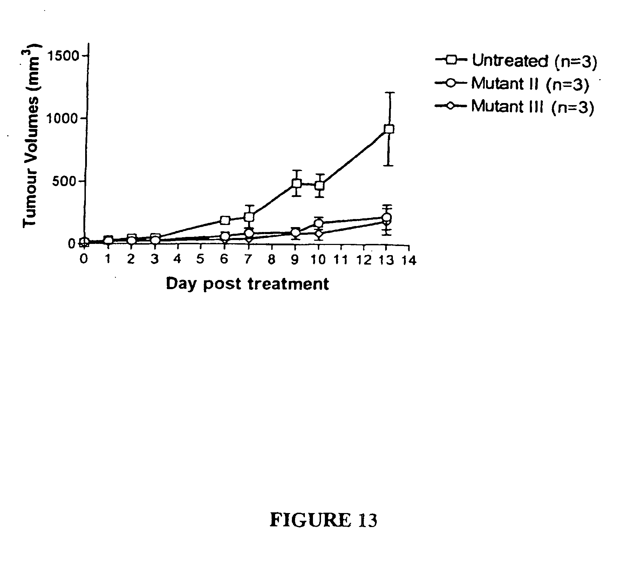

SK-Mel3 human melanoma xenografₗs were established in 5-6 week old CD-I athymic mice. On day 0, tumours were either left untreated or were treated intravenously with 5 x10⁹ pfu of mutant VSV as indicated. Results are shown in Figure 13.

This example demonstrates that mutants II and III are able to inhibit tumour growth following a single intravenous injection. Thus virus need not be administered at the tumour site to be effective in inhibiting tumour growth. Furthermore, the mutants while being attenuated for growth in normal mouse tissues. are still able to target tumour cells *in vivo.*

### Example 25. Selective killing of AML Cells Co-cultured with normal bone marrow.

The growth factor independent cell line OCI/AML3 was mixed 1:9 with normal bone marrow and infected for 24 hours with WT Indiana VSV. Various dilutions of cells were then plated in methylcellulose plus and minus growth factors and colony counts were performed 14 days later. Table 10 shows data for dishes receiving 10⁴ cells. The asterisk (*) signifies that no leukemic colonies were detected on the growth factor minus dishes even when 10⁵ cells were plated per dish.

This example demonstrates the rapid and selective killing of leukemia cells in the presence of normal bone stem cells. Furthermore it demonstrates that bone marrow is not a dose-limiting target of VSV oncolytic therapy as it is with most other conventional cancer therapies.

**TABLE 10**

| | **Multiplicity of Infection** | | |
|---|---|---|---|
| **Colony Type** | **0.0** | **1.0** | **5.0** |
| | | | |
| Leukemic | 172 | 0* | 0* |
| Neutrophil | 12 | 7 | 5 |
| Mixed | 6 | 3 | 4 |
| Monocyte | 10 | 7 | 5 |

### Example 26. VSV Sequences

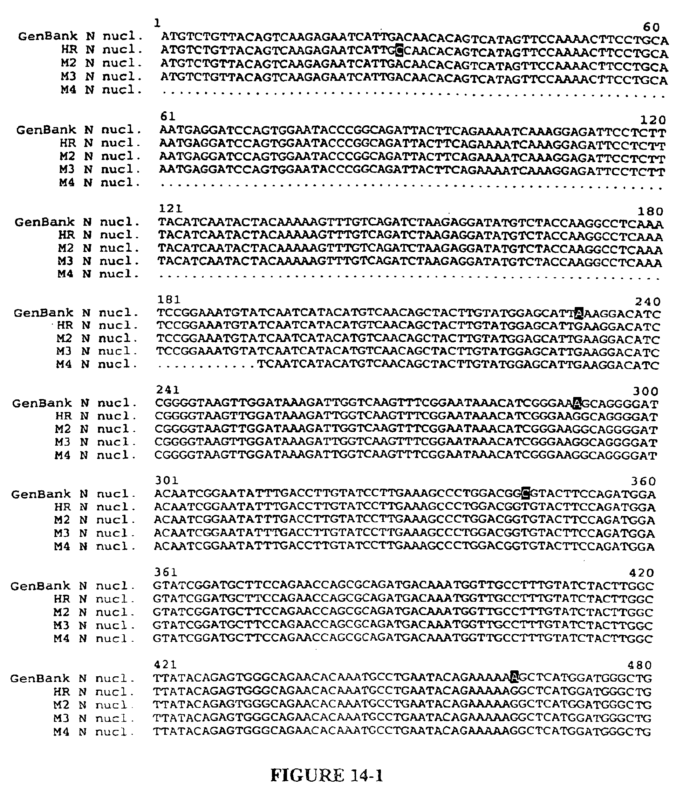

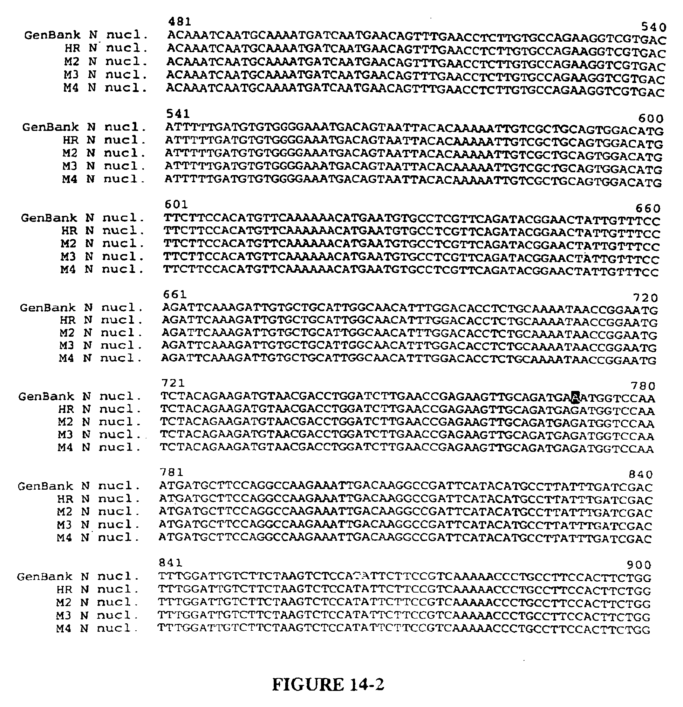

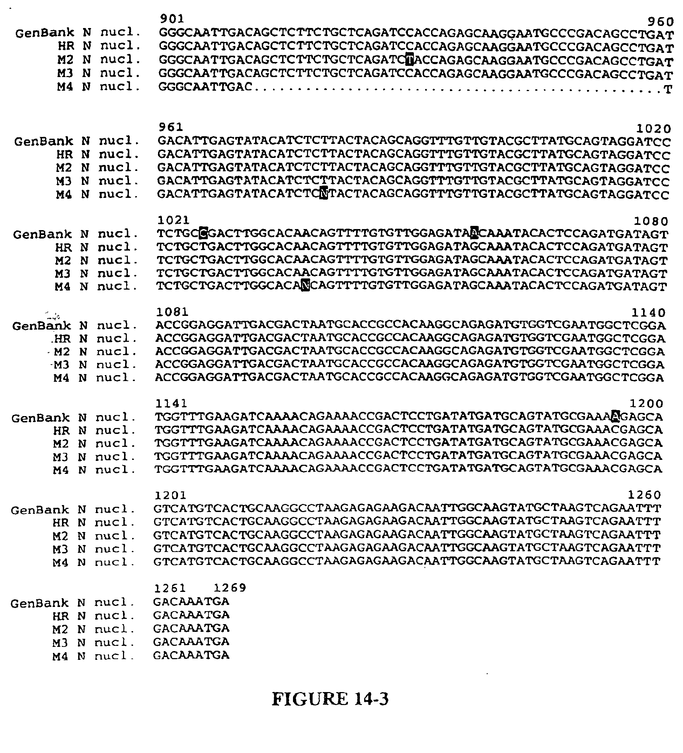

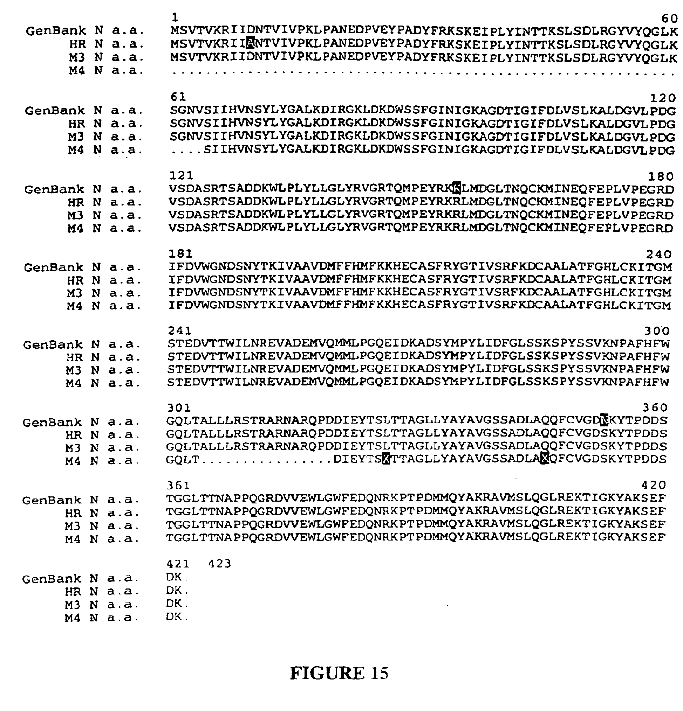

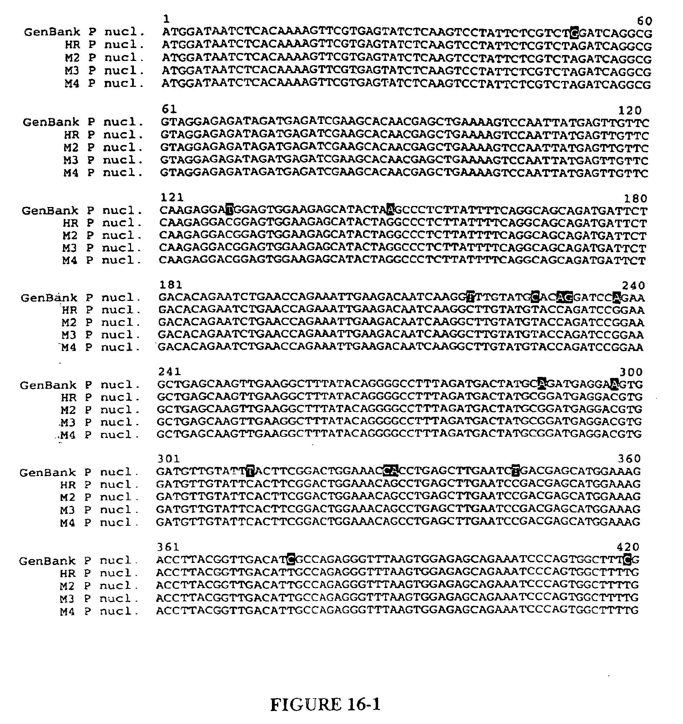

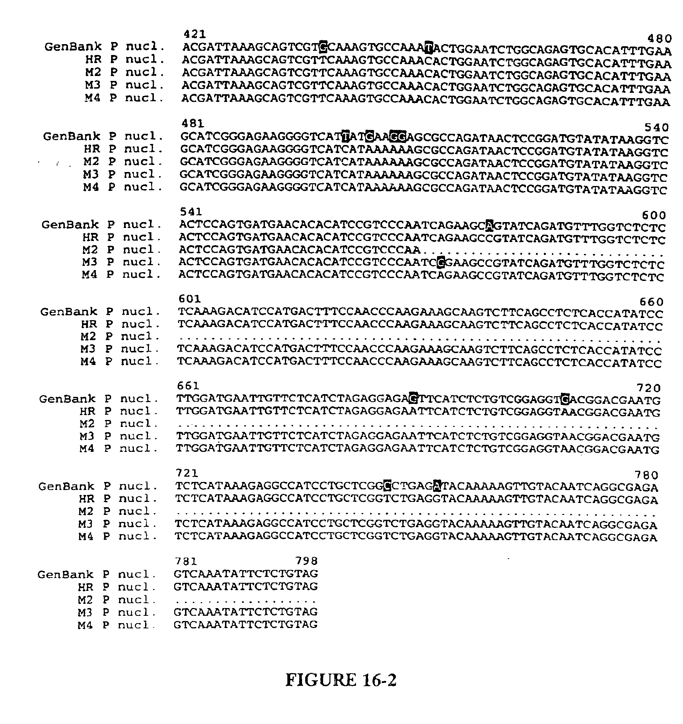

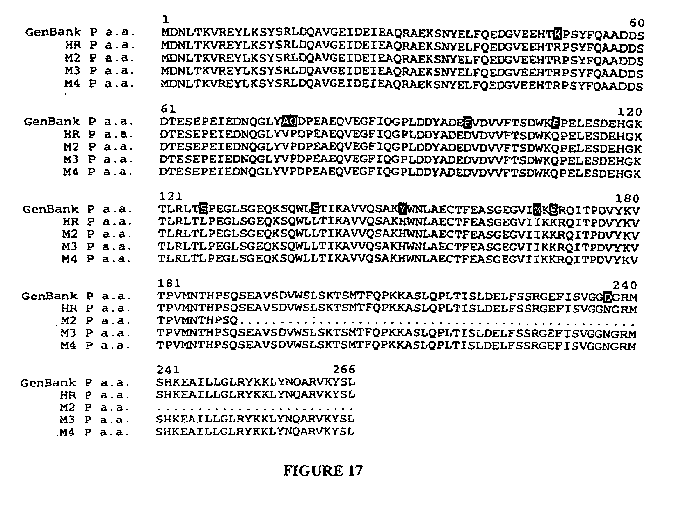

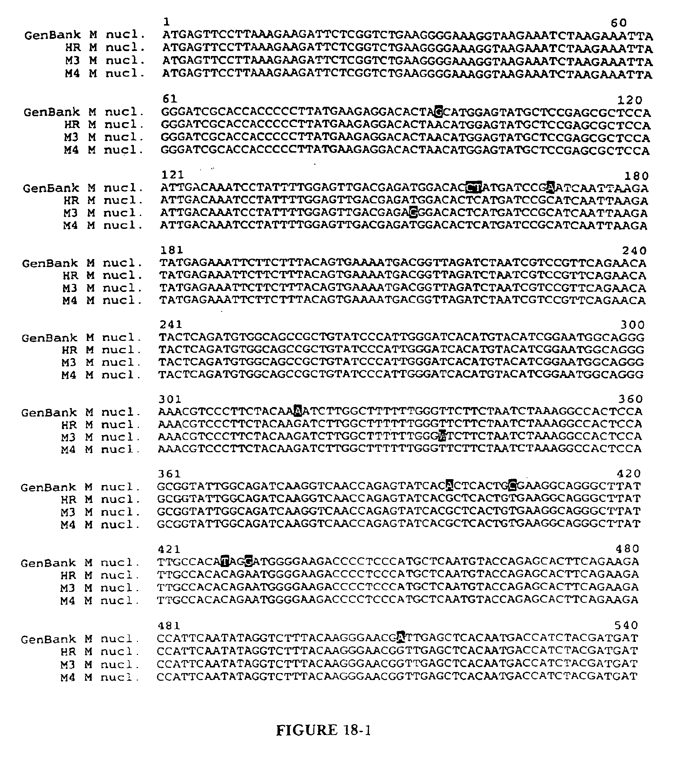

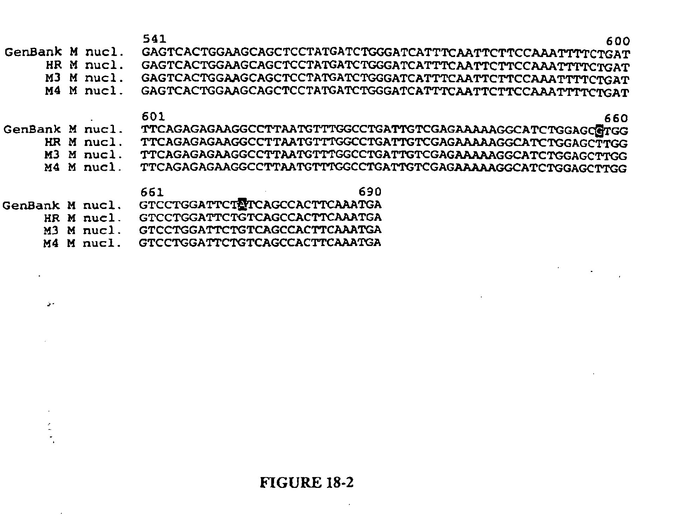

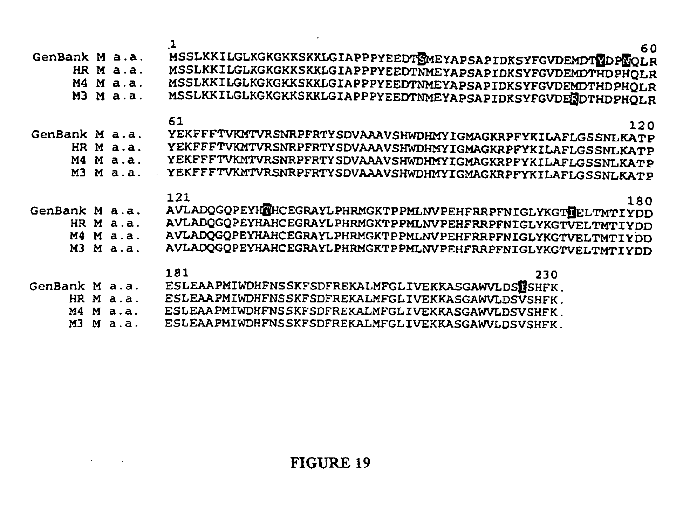

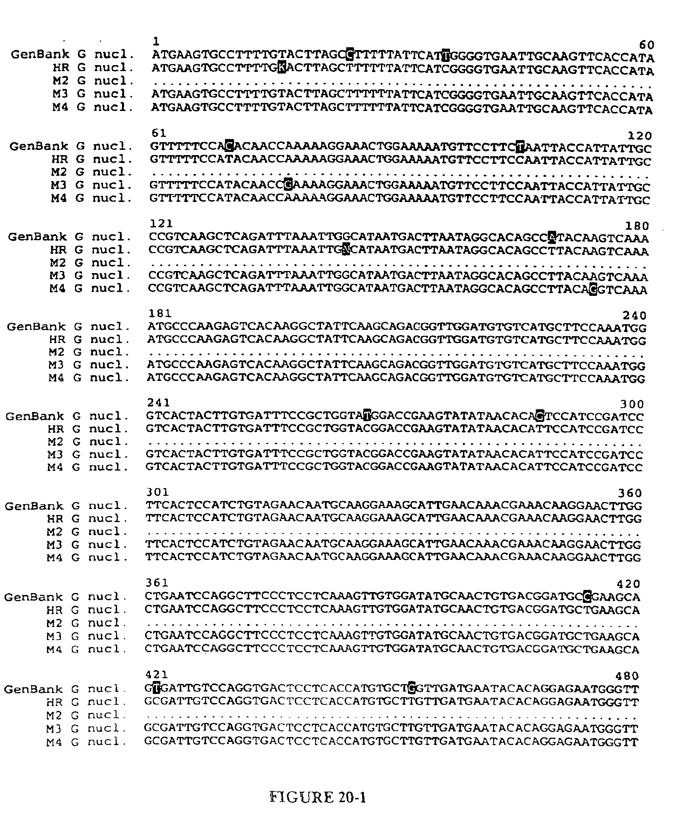

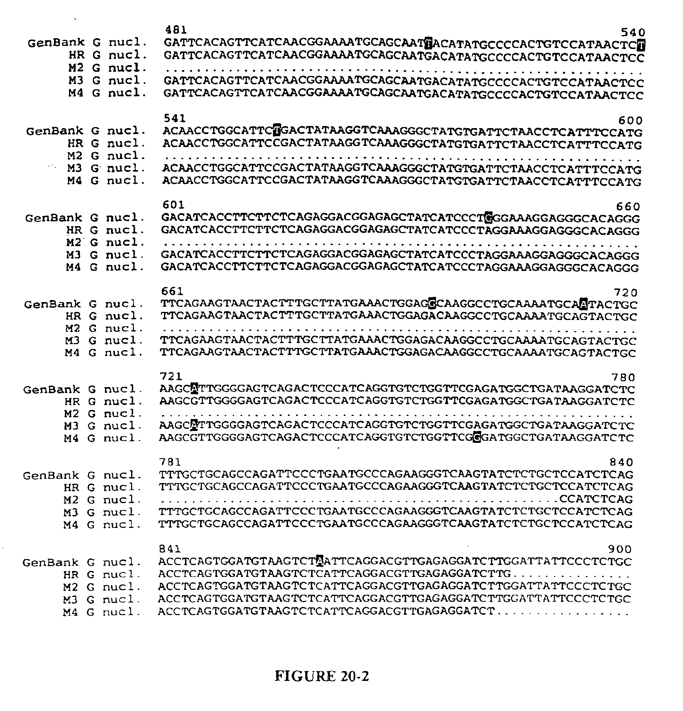

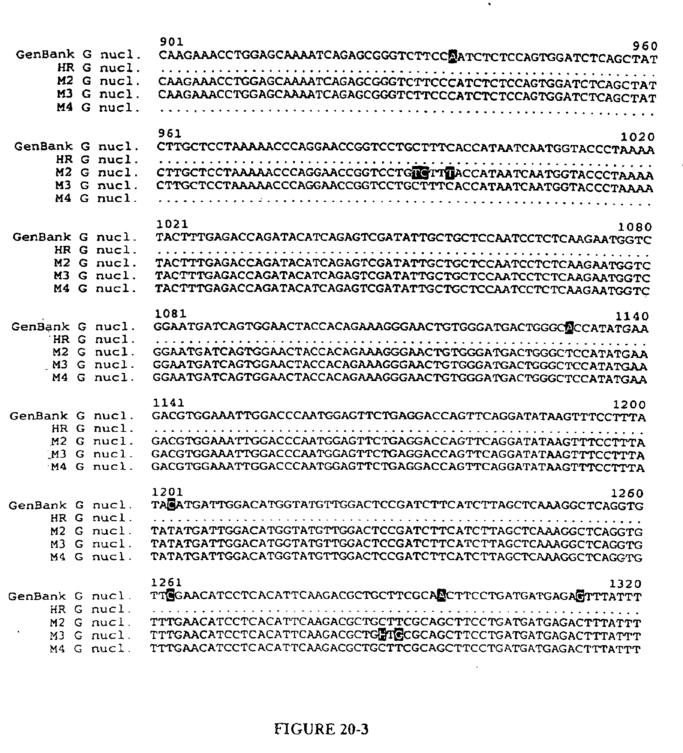

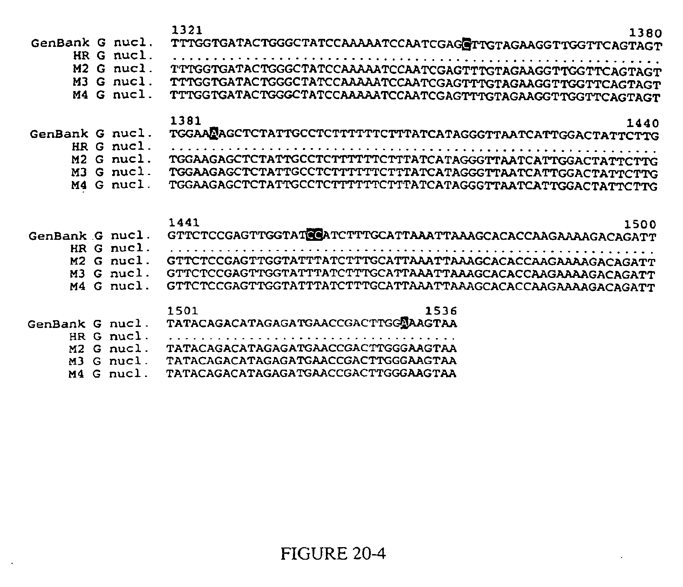

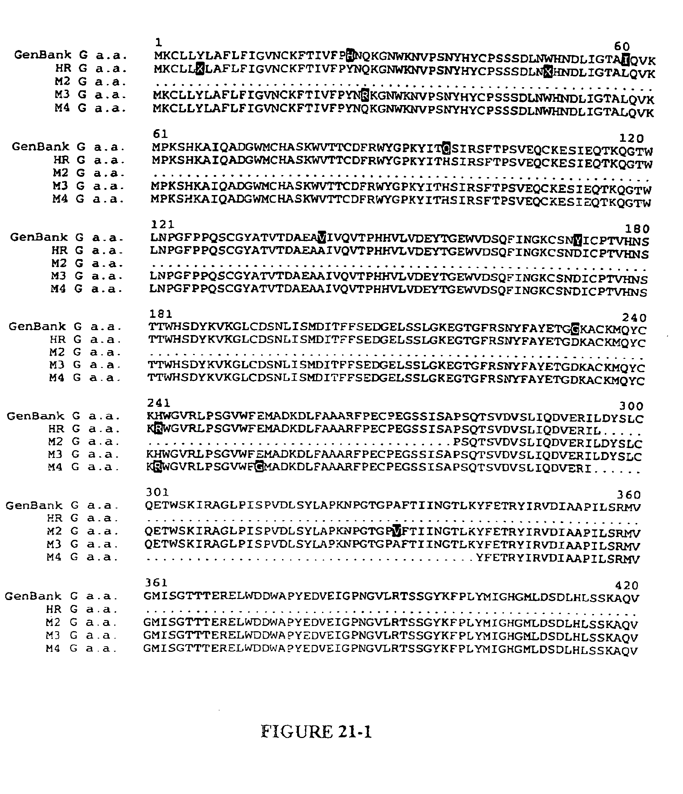

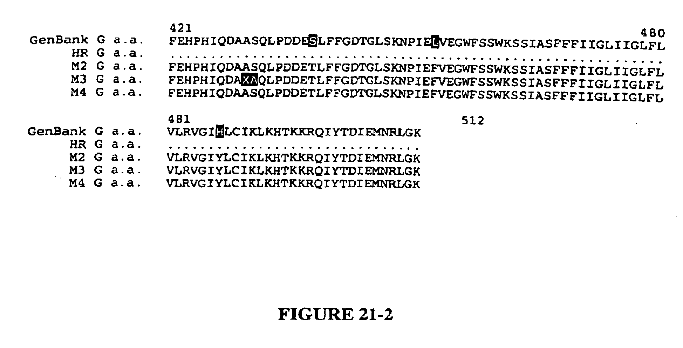

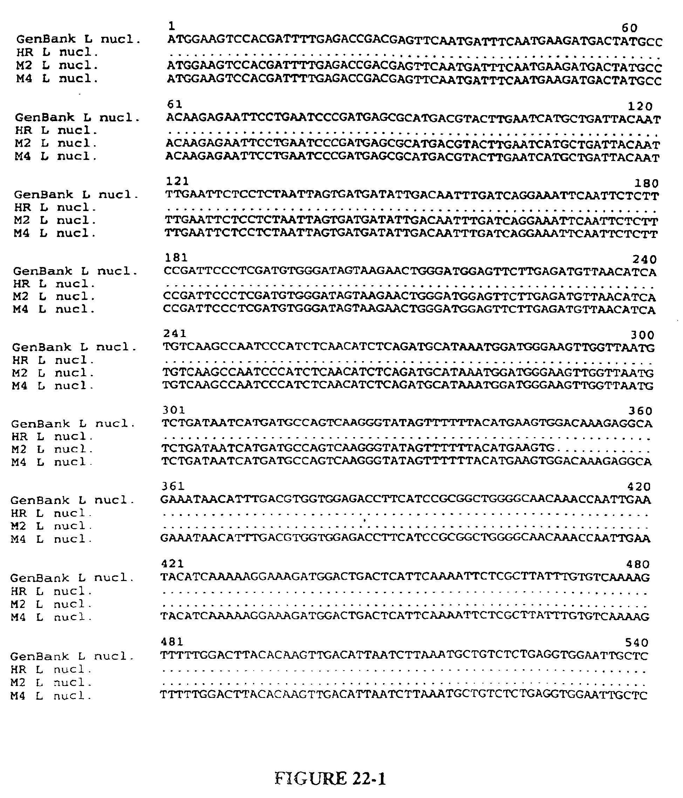

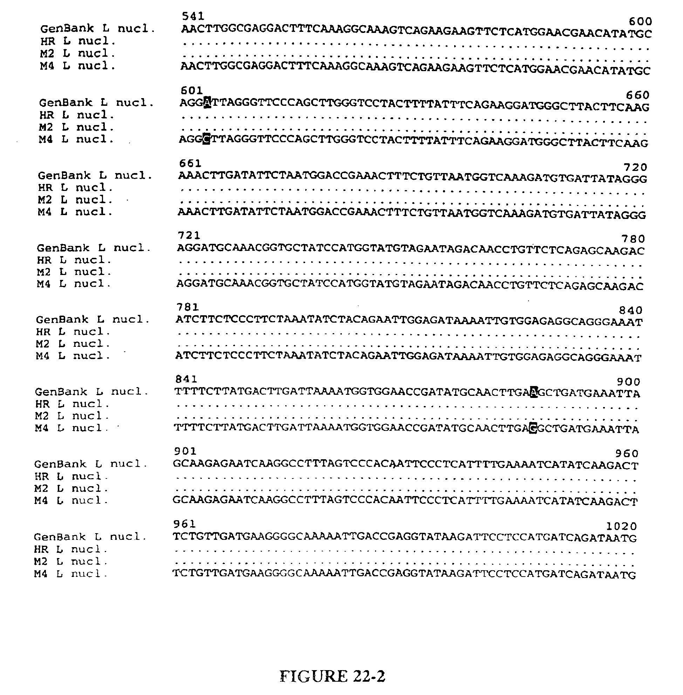

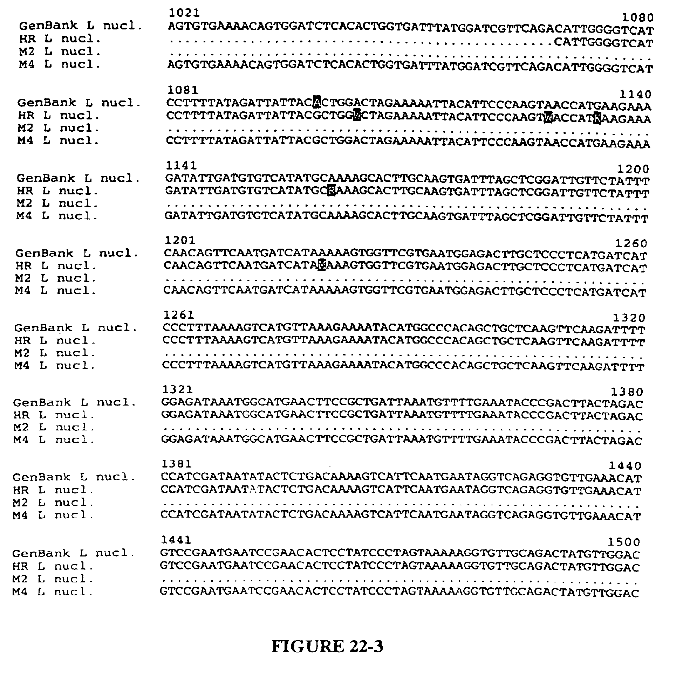

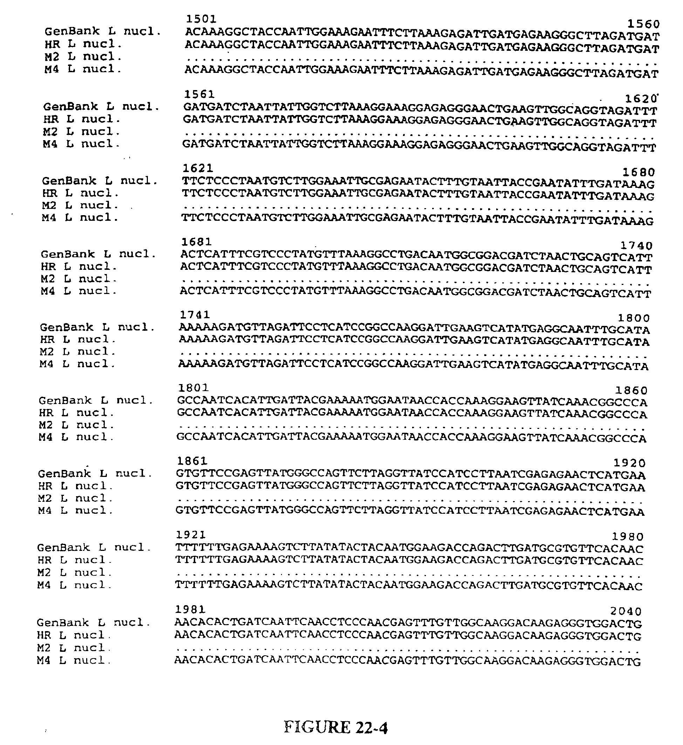

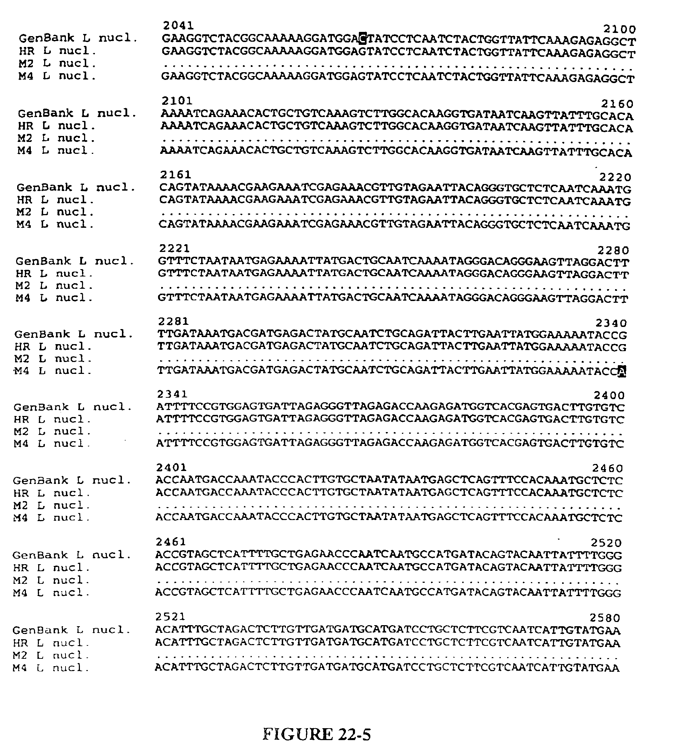

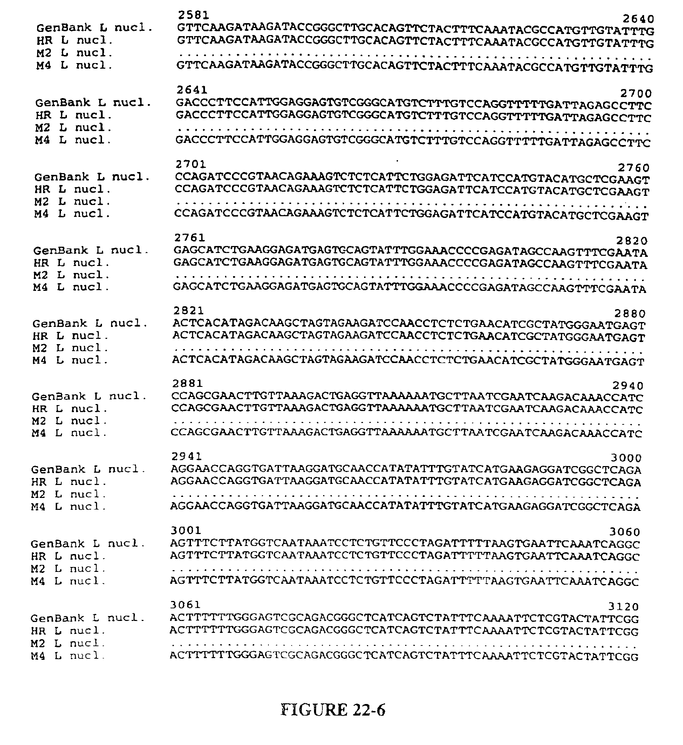

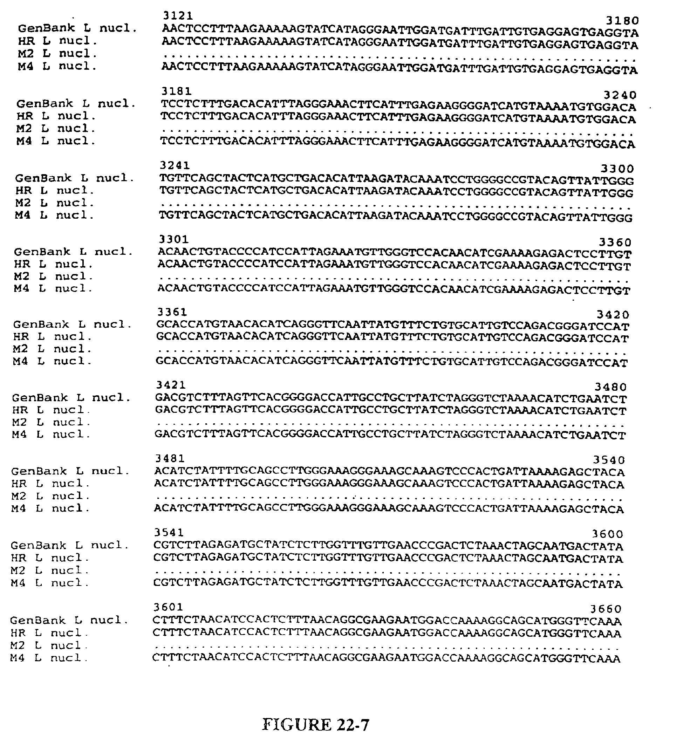

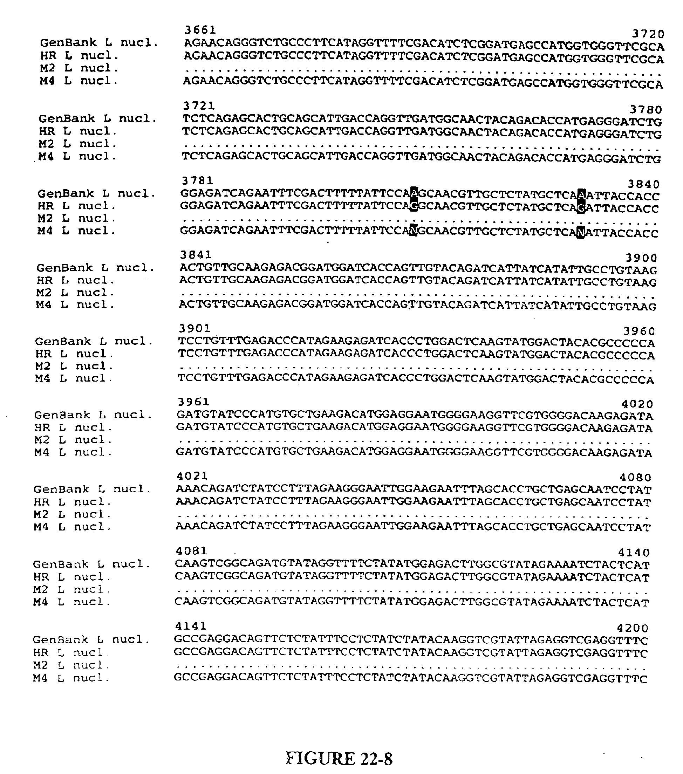

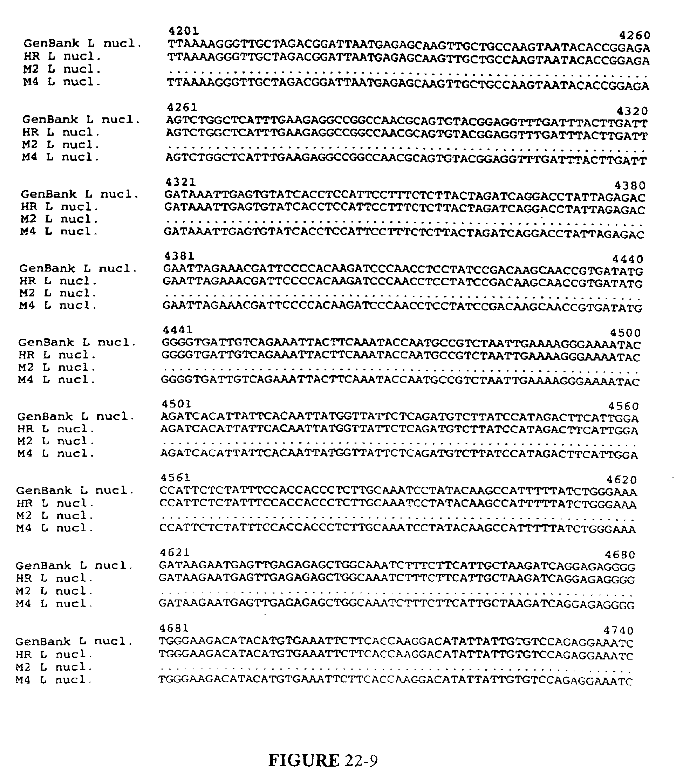

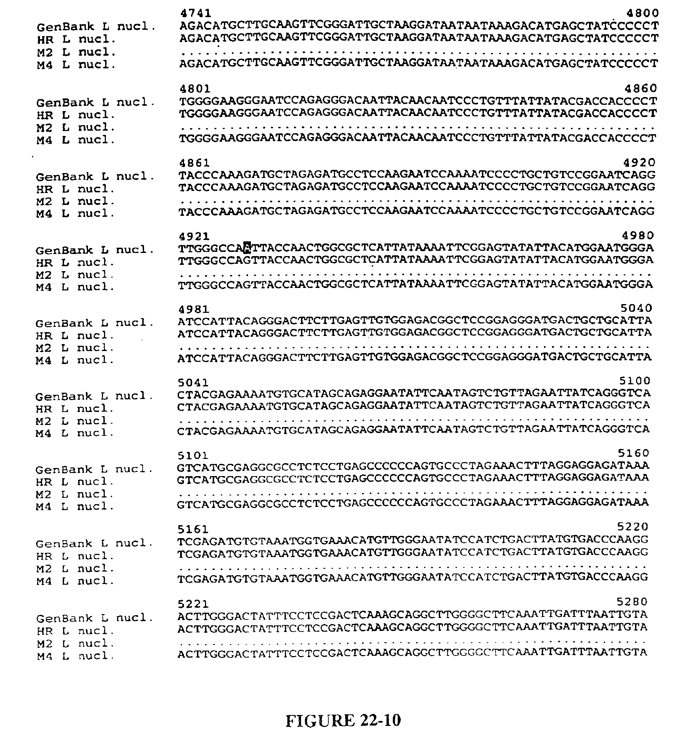

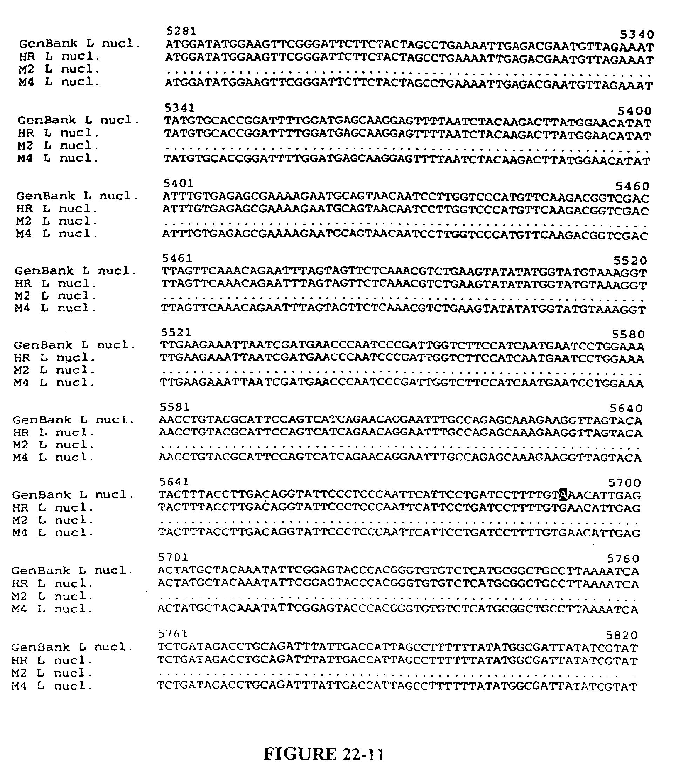

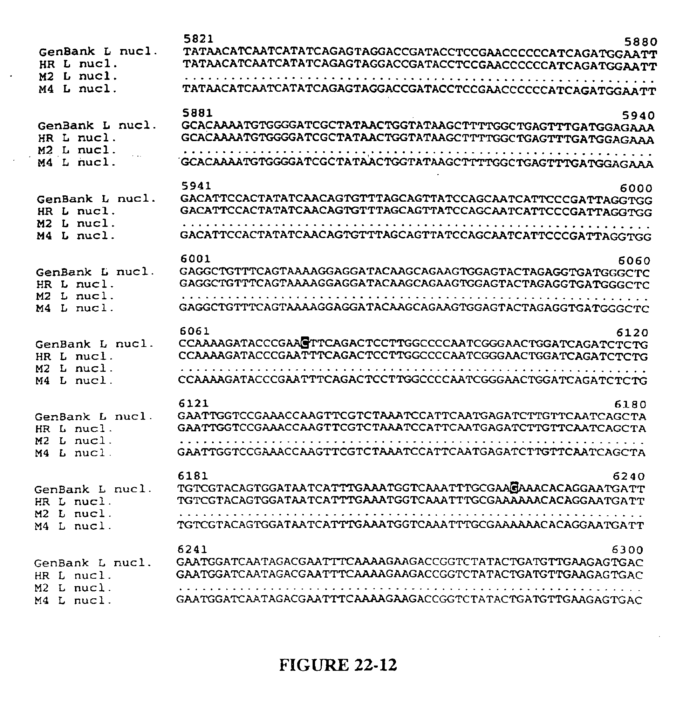

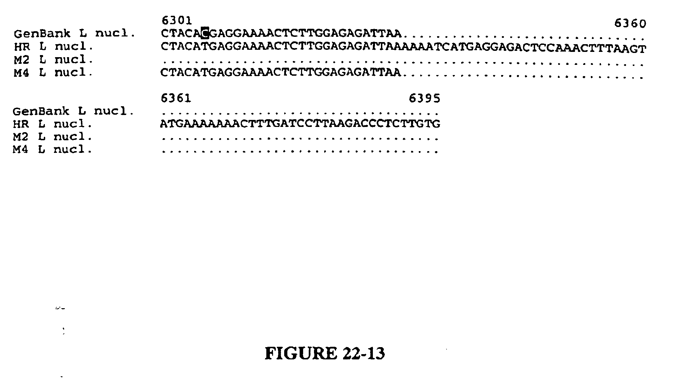

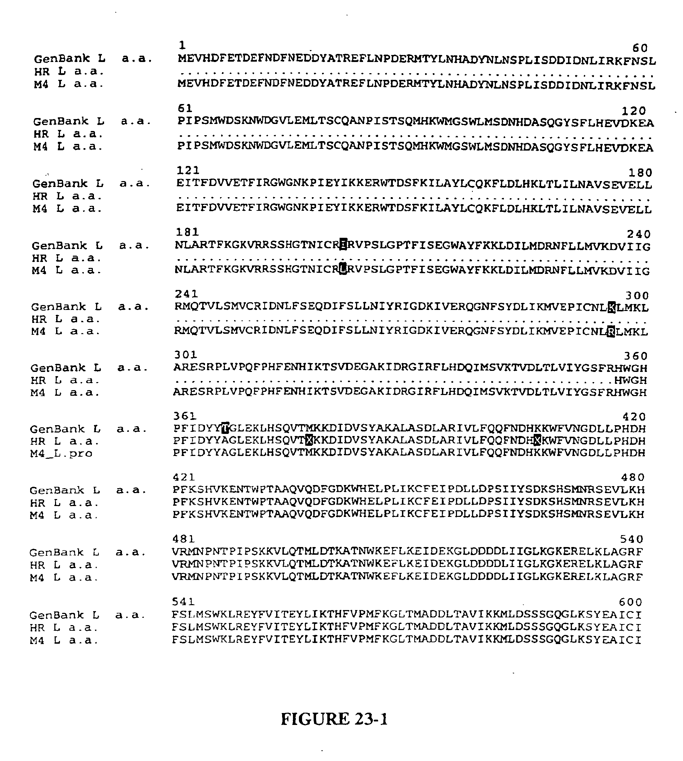

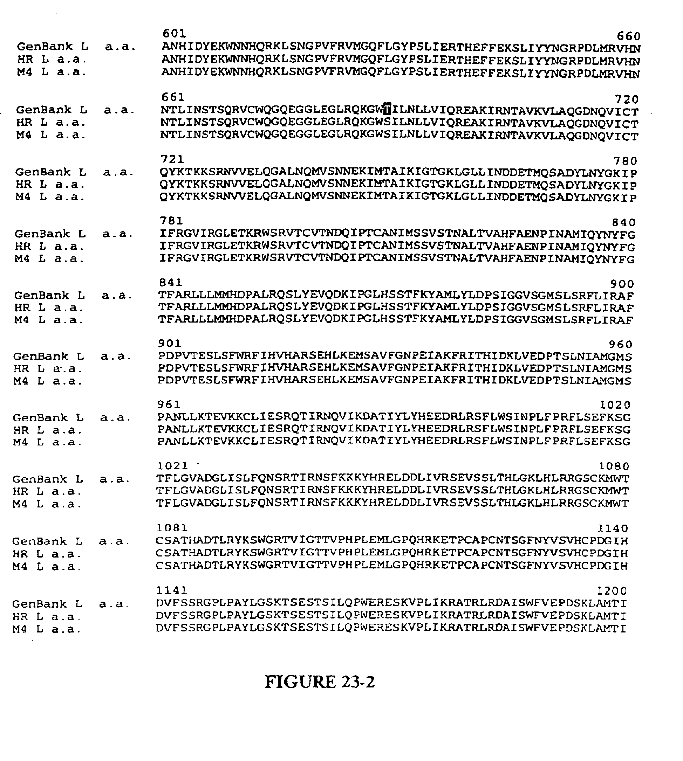

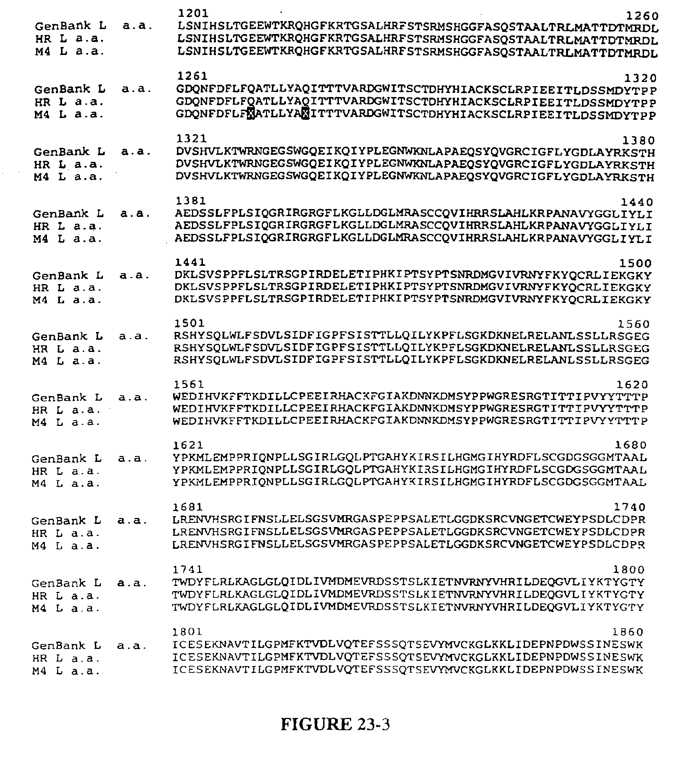

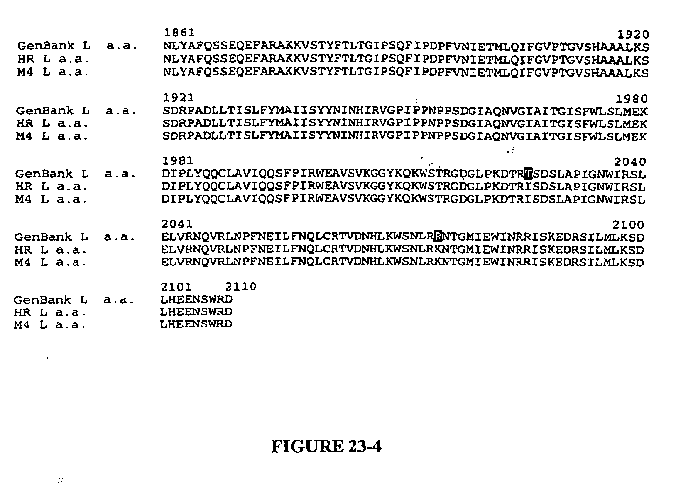

The genome of VSV contains genes that encode viral proteins N. P. M. G and L. The cDNA sequences of the open reading frames (ORF) for these proteins from wild type heat resistant VSV (HR) and three mutant VSVs were determined (based on sequencing five times each) and compared with the sequences of GenBank Accession No. NC 001560 (derived from Colorado and San Juan strains of VSV). The mutants are M2 (TR 1026R), M3 (TP3) and M4 (TP6). The nucleic acid sequences are shown in Figures 14, 16, 18, 20 and 22. The corresponding deduced amino acid sequences are shown in Figures 15, 17, 19, 21 and 23, respectively. Differences are indicated by highlighted letters. Dotted lines represent incomplete sequencing.

Some of the differences between the amino acid sequences are shown in Table I I using notation based on column heading (i.e. for column heading "Differences Between GenBank and HR" notation K155R means that the amino acid at position 155 is K in GenBank and R in HR). In those cases where HR sequence is not yet available comparisons can only be made between GenBank and a particular mutant. M3* denotes a difference between mutant 3 and HR but in this case the amino acid matches the GenBank deposit at that position (i.e. mutant 3 and the Genbank sequence agree at that position white HR is different).

This data demonstrates the many differences in sequence between the HR strain and the GenBank deposit (which is primarily derived from the San Juan strain). It also demostrates some of the differences between the mutants and the HR strain from which they were derived. These genetic differences correlate with phenotypic differences.

**TABLE 11**

| Gene | Differences Between GenBank and HR | Differences Between HR and Mutants | Differences Between Genbank and Mutant #2 | Differences Between GenBank and Mutant #4 |
|---|---|---|---|---|
| N | D10A, K155R, N353S | A10D (M3*) None (M4) | | |
| P | K50R, A76V, Q77P, E99D P110Q, S126L, S140L Y151H, M168I, E170K D237N | None (M2, M3 and M4) | | |
| M | S32N. Y54H. N57H, T133A, 1171V, I226V | M51R (M3) None (M4) | | |
| G | H24Y, I57L, Q96H, V141A, Y172D, G132D H242R, S438T, L453F H487Y | Q26R, R242H, S431A (all M3) E254G (M4) | A331V | |
| L | T367A, T689S, T20261 | None (M4) | | I202L, K296R |
| | R2075K | | | |

### References

1. Stark, G.R.. et al., How cells respond to interferons. Annu Rev Biochem, 1998. 67: p. 227-64.
2. Jaramillo, M.L., N. Abraham, and J.C. Bell, The interferon system: a review with emphasis on the role of PKR in growth control. Cancer Invest, 1995. 13(3): p. 327-38.
3. Cuddihy, A.R., et al., Double-stranded-RNA-activated protein kinase PKR enhances transcriptional activation by tumor suppressor p53. Mol Cell Biol, 1999. 19(4): p. 2475-84.
4. Lee. S.B., et al., The apoptosis pathway triggered by the interferon-induced protein kinase PKR requires the third basic domain, initiates upstream of Bcl-2, and involves ICE-like proteases. Virology, 1997.231(1): p. 81-8.
5. Lee, S.B. and M. Esteban. The interferon-induced double-stranded RNA-activated protein kinase induces apoptosis. Virology. 1994. 199(2): p. 491-6.
6. Gale, M.J., Jr., et al., Evidence that hepatitis C virus resistance to interferon is mediated through repression of the PKR protein kinase by the nonstructural 5A protein. Virology, 1997. 230(2): p. 217-27.
7. McMillan, N.A., et al., HIV-1 Tat directly interacts with the interferon-induced, double- stranded RNA-dependent kinase, PKR. Virology, 1995. 213(2): p. 413-24.
8. Kitajewski, J., et al., Adenovirus VAI RNA antagonizes the antiviral action of interferon by preventing activation of the interferon-induced eIF-2 alpha kinase. Cell, 1986. 45(2): p. 195-200.
9. Black, T.L., G.N. Barber, and M.G. Katze, Degradation of the interferon-induced 68,000-M(r) protein kinase by polio virus requires RNA. J Virol, 1993. 67(2): p. 791-800.
10. Melville, M.W., et al., The molecular chaperone hsp40 regulates the activity of P58IPK, the cellular inhibitor of PKR. Proc Natl Acad Sci U S A, 1997. 94(1): p. 97-102.
11. Swaminathan, S., et al., Simian Virus 40 Large-T Bypasses the Translational Block Imposed by the Phosphorylation of eIF-2alpha. Virology, 1996, 219(1): p. 321-3.
12. Der, S.D., et al., A double-stranded RNA-activated protein kinase-dependent pathway mediating stress-induced apoptosis. Proc Natl Acad Sci U S A, 1997. 94(7): p. 3279-83.
13. Meurs, E.F., et al., Tumor suppressor function of the interferon-induced double-stranded RNA- activated protein kinase. Proc Natl Acad Sci U S A, 1993 90(1): p. 232-6.
14. Koromilas, A.E., et al., Malignant transformation by a mutant of the IFN-inducible dsRNA- dependent protein kinase. Science, 1992. 257(5077): p. 1685-9.
15. Mundschau, L.J. and D.V. Faller, Oncogenic ras induces an inhibitor of double-stranded RNA-dependent eukaryotic initiation factor 2 alpha-kinase activation. J Biol Chem, 1992. 267(32): p. 23092-8.
16. Mundschau, L.J. and D.V. Faller, Endogenous inhibitors of the dsRNA-dependent eIF-2 alpha protein kinase PKR in normal and ras-transformed cells. Biochimie, 1994. 76(8): p. 792-800.
17. Strong, J.E., et al., The molecular basis of viral oncolysis: usurpation of the Ras signalling pathway by reovirus. Embo J, 1998. 17(12): p. 3351-62.
18. Kumar, K.U., S.P. Srivastava, and R.J. Kaufman, Double-stranded RNA-activated protein kinase (PKR) is negatively regulated by 60S ribosomal subunit protein L18. Mol Cell Biol, 1999. 19(2): p. 1116-25.
19. Peralta, R.C., et al., Distinct regions of frequent loss of heterozygosity of chromosome 5p and 5q in human esophageal cancer. Int J Cancer, 1998. 78(5): p. 600-5.
20. Kirchhoff, S., et al., IRF-1 induced cell growth inhibition and interferon induction requires the activity of the protein kinase PKR. Oncogene, 1995. 11(3): p. 439-45.
21. Beretta, L., et al., Expression of the protein kinase PKR in modulated by IRF-1 and is reduced in 5q- associated leukemias. Oncogene, 1996. 12(7): p. 1593-6.
22. Lossos, I.S., et al., A novel translocation (1;2)(p34;p21-22) in acute myelomonoblastic leukemia. Cancer Genet Cytogenet, 1998. 106(1): p. 78-9.
23. Haines, G.K.d., et al., Expression of the double-stranded RNA-dependent protein kinase (p68) in squamous cell carcinoma of the head and neck region. Arch Otolaryngol Head Neck Surg, 1993. 119(10): p. 1142-7.
24. Haines, G.K., et al., Correlation of the expression of double-stranded RNA-dependent protein kinase (p68) with differentiation in head and neck squamous cell carcinoma. Virchows Arch B Cell Pathol Incl Mol Pathol, 1993, 63(5): p. 289-95.
25. Shimada, A., et al., Aberrant expression of double-stranded RNA-dependent protein kinase in hepatocytes of chronic hepatitis and differentiated hepatocellular carcinoma. Cancer Res, 1998. 58(19): p. 4434-8.
26. Wong, L.H., et al., Interferon-resistant human melanoma cells are deficient in ISGF3 components, STAT1, STAT2, and p48-ISGF3gamma. J Biol Chem, 1997, 272(45): p. 28779-85.
27. Petricoin, E., 3rd, et al., Human cancer cell lines express a negative transcriptional regulator of the interferon regulatory factor family of DNA binding proteins. Mol Cell Biol. 1994. 14(2): p. 1477-86.
28. Abril, E., et al., Characterization of a gastric tumor cell line defective in MHC class I inducibility by both alpha- and gamma-interferon. Tissue Antigens, 1996. 47(5): p. 391-8.
29. Abril, E., et al., Unresponsiveness to interferon associated with STAT1 protein deficiency in a gastric adenocarcinoma cell line. Cancer Immunol Immunother, 1998. 47(2): p. 113-20.
30. Sun, W.H., et al., Interferon-alpha resistance in a cutaneous T-cell lymphoma cell line is associated with lack of STAT1 expression. Blood, 1998. 91(2): p. 570-6.
31. Chelbi-Alix, M.K., et al., Resistance to virus infection conferred by the interferon-induced promyelocytic leukemia protein. J Virol, 1998. 72(2): p. 1043-51.
32. Chelbi-Alix, M.K., et al., Induction of the PML protein by interferons in normal and APL cells. Leukemia, 1995. 9(12): p. 2027-33.
33. Stadler, M., et al., Transcriptional induction of the PML growth suppressor gene by interferons is mediated through an ISRE and a GAS element. Oncogene, 1995. 11(12): p. 2565-73.
34. Koken, M.H., et al., Leukemia-associated retinoic acid receptor alpha fusion partners, PML and PLZF, heterodimerize and colocalize to nuclear bodies. Proc Natl Acad Sci U S A. 1997. 94(19): p. 10255-60.
35. Abraham, N., et al., Characterization of transgenic mice with targeted disruption of the catalytic domain of the double-stranded RNA-dependent protein kinase, PKR. J Biol Chem, 1999. 274(9): p. 5953-62.
36. Garcia-Sastre, A., et al., Influenza A virus lacking the NS 1 gene replicates in interferon- deficient systems. Virology, 1998. 252(2): p. 324-30.

## Claims

1. Use of a virus for the manufacture of a medicament for reducing the viability of a tumor cell, wherein the virus is an attenuated strain of vesicular stomatitis virus and wherein the tumor cell is a hematopoietic cancer cell, a melanoma, a sarcoma, a neuroendocrine tumor, a lung carcinoma or a colon carcinoma.

2. Use according to Claim 1, wherein the tumor cell is a lung carcinoma.

3. Use according to Claim 1, wherein the tumor cell is a hematopoietic cancer cell.

4. Use according to Claim 3, wherein the hematopoietic cancer cell is a leukemia, a lymphoma, or a myeloma.

5. Use according to Claim 4, wherein the hematopoietic cancer cell is a leukemia.

6. Use according to Claim 5, wherein the leukemia is acute myelogenous leukemia.

7. Use according to Claim 5, wherein the leukemia is chronic myelogenous leukemia.

8. Use according to Claim 5, wherein the leukemia is promyelocytic leukemia.

9. Use according to Claim 5, wherein the leukemia is T cell leukemia.

10. Use according to Claim 4, wherein the hematopoietic cancer cell is a lymphoma.

11. Use according to Claim 4, wherein the hematopoietic cancer cell is a myeloma.

12. Use according to Claim 1, wherein the tumor cell is a sarcoma.

13. Use according to Claim 12, wherein the sarcoma is an osteosarcoma.

14. Use according to Claim 12, wherein the sarcoma is a fibrosarcoma.

15. Use according to Claim 1, wherein the tumor cell is neuroendocrine tumor.

16. Use according to Claim 1, wherein the tumor cell has substantial no PKR activity.

17. Use according to Claim 1, wherein the tumor cell is PKR-/-; STAT1-/-; or both PKR-/- and STAT1-/-.

18. Use according to Claim 5 further comprising administering interferon to the tumor cell prior to administering VSV.

19. Use according to Claim 1, wherein the virus is vesicular stomatitis virus strain M1.

20. Use according to Claim 1, wherein the virus is vesicular stomatitis virus strain M2.

21. Use according to Claim 1, wherein the virus is vesicular stomatitis virus strain M3.

22. Use according to Claim 1, wherein the virus is vesicular stomatitis virus strain M4.

23. Use according to Claim 1, wherein the virus is vesicular stomatitis virus strain M5.

24. Use according to Claim 1, wherein the tumor cell is in a mammalian subject and the virus is for administration to the tumor cell by intravenous, intranasal, intraperitoneal or intratumoral administration to the subject.

25. Use according to Claim 24, wherein the mammalian subject is a human or non-human mammal.

26. Use according to Claim 24, wherein the virus is contained in cell line infected with the virus and the administration comprises administering the virus-infected cell line to the subject by a route selected from intratumorally, intravenously or intraperitoneally.

27. Use according to Claim 1, wherein the virus has an N gene nudeic acid sequence that codes for the same N protein amino acid sequence as the N gene cDNA sequence for vesicular stomatitis virus strain M2, M3, or M4, as shown in Figure 14.

28. Use according to Claim 1, wherein the virus has a P gene nucleic acid sequence that codes for the same P protein amino acid sequence as the P gene cDNA sequence for vesicular stomatitis virus strain M2, M3, or M4, as shown in Figure 16.

29. Use according to Claim 1, wherein the virus has a M gene nucleic acid sequence that codes for the same M protein amino acid sequence as the M gene cDNA sequence for vesicular stomatitis virus strain M3 or M4, as shown in Figure 18.

30. Use according to Claim 1, wherein the virus has a G gene nucleic acid sequence that codes for the same G protein amino acid sequence as the G gene cDNA sequence for vesicular stomatitis virus strain M2, M3, or M4, as shown in Figure 20.

31. Use according to Claim 1, wherein the virus has a L gene nucleic acid sequence that codes for the same L protein amino acid sequence as the L gene cDNA sequence for vesicular stomatitis virus strain M2 or M4, as shown in Figure 22.

32. An isolated nucleic acid molecule having a sequence comprising:
(a) a sequence coding for a protein comprising the amino acid sequence of Protein N of VSV strain HR shown in Figure 15; or
(b) N gene cDNA sequence of a VSV strain selected from the group consisting of HR, M2 and M3 shown in Figure 14; or
(c) RNA sequence corresponding to (b); or
(d) a sequence complementary to (a), (b) or (c).

33. An isolated nucleic acid molecule having a sequence comprising:
(a) a sequence coding for a protein comprising the amino acid sequence of Protein P of VSV strain HR, shown in Figure 17; or
(b) P gene cDNA sequence of a VSV strain selected from the group consisting of HR and M3, shown in Figure 16; or
(c) RNA sequence corresponding to (b); or
(d) a sequence complementary to (a), (b) or (c).

34. An isolated nucleic acid molecule having a sequence comprising:
(a) a sequence coding for a protein comprising the amino acid sequence of Protein M of a VSV strain selected from the group consisting of HR, M3 and M4 shown in Figure 19; or
(b) M gene cDNA sequence of a VSV strain selected from the group consisting of HR, M3 and M4 shown in Figure 18; or
(c) RNA sequence corresponding to (b); or
(d) a sequence complementary to (a), (b) or c.

35. An isolated nucleic acid molecule having a sequence comprising:
(a) a sequence coding for a protein comprising the amino acid sequence of Protein G of VSV strain M3 shown in Figure 21; or
(b) G gene cDNA sequence of VSV strain M3 shown in Figure 20; or
(c) RNA sequence corresponding to (b); or
(d) a sequence complementary to (a), (b) or (c).

36. An isolated nucleic acid molecule having a sequence comprising:
(a) a sequence coding for a protein comprising the amino acid sequence of Protein L of VSV strain M4 shown in Figure 23; or
(b) L gene cDNA sequence of VSV strain M4 shown in Figure 22 or
(c) RNA sequence corresponding to (b); or
(d) a sequence complementary to (a), (b) or (c).

## Patentansprüche

1. Verwendung eines Virus zur Herstellung eines Medikaments zur Reduktion der Lebensfähigkeit einer Tumorzelle, wobei das Virus ein attenuierter Stamm des vesikulären Stomatitis-Virus ist und wobei die Tumorzelle eine hämatopoietische Krebszelle, ein Melanom, ein Sarkom, ein neuroendokriner Tumor, ein Lungenkarzinom oder ein Colon-Karzinom ist.

2. Verwendung nach Anspruch 1, wobei die Tumorzelle ein Lungenkarzinom ist.

3. Verwendung nach Anspruch 1, wobei die Tumorzelle eine hämatopoietische Krebszelle ist.

4. Verwendung nach Anspruch 3, wobei die hämatopoietische Krebszelle eine Leukämie, ein Lymphom oder ein Myelom ist.

5. Verwendung nach Anspruch 4, wobei die hämatopoietische Krebszelle Leukämie ist.

6. Verwendung nach Anspruch 5, wobei die Leukämie akute myelogene Leukämie ist.

7. Verwendung nach Anspruch 5, wobei die Leukämie chronische myelogene Leukämie ist.

8. Verwendung nach Anspruch 5, wobei die Leukämie promyelozytische Leukämie ist.

9. Verwendung nach Anspruch 5, wobei die Leukämie eine T-Zell-Leukämie ist.

10. Verwendung nach Anspruch 4, wobei die hämatopoietische Krebszelle ein Lymphom ist.

11. Verwendung nach Anspruch 4, wobei die hämatopoletische Krebszelle ein Myelom ist.

12. Verwendung nach Anspruch 1, wobei die Tumorzelle ein Sarkom ist.

13. Verwendung nach Anspruch 12, wobei das Sarkom ein Osteosarkom ist.

14. Verwendung nach Anspruch 12, wobei das Sarkom ein Fibrosarkom ist

15. Verwendung nach Anspruch 1, wobei die Tumorzelle ein neuroendokriner Tumor ist.

16. Verwendung nach Anspruch 1, wobei die Tumorzelle im wesentlichen keine PKR-Aktivität aufwelst.

17. Verwendung nach Anspruch 1, wobei die Tumorzelle PKR-/-, STAT1-/- oder sowohl PKR-/- und STAT1-/- ist.

18. Verwendung nach Anspruch 5, wobei die Verwendung weiterhin die Verabreichung von Interferon an die Tumorzelle vor der Verabreichung von VSV umfasst.

19. Verwendung nach Anspruch 1, wobei das Virus der vesikuläre Stomatitis-Virus-Stamm M1 ist.

20. Verwendung nach Anspruch 1, wobei das Virus der vesikuläre Stomatitis-Virus-Stamm M2 ist.

21. Verwendung nach Anspruch 1, wobei das Virus der vesikuläre Stomatitis-Virus-Stamm M3 ist.

22. Verwendung nach Anspruch 1, wobei das Virus der vesikuläre Stomatitis-Virus-Stamm M4 ist.

23. Verwendung nach Anspruch 1, wobei das Virus der vesikuläre Stomatitis-Virus-Stamm M5 ist.

24. Verwendung nach Anspruch 1, wobei dle Tumorzelle In einem Säugetier-Lebewesen auftritt und das Virus für die Verabreichung an die Tumorzelle durch intravenöse, intranasale, intraperitoneale oder intratumorale Verabreichung an das Lebewesen geeignet ist

25. Verwendung nach Anspruch,24, wobei das Säugetier-Lebewesen ein Mensch oder ein nicht-humanes Säugetier ist.

26. Verwendung nach Anspruch 24, wobei das Virus in einer mit dem Virus infizierten Zelllinie enthalten ist und die Verabreichung die Verabreichung der Virusinfizierten Milinie an das Lebewesen auf einem Weg, ausgewählt aus intratumoraler, intravenöser oder intraperitonealer Verabreichung, umfasst.

27. Verwendung nach Anspruch 1, wobei das Virus eine N-Cen-Nukleinsäuresequenz aufweist, die für dieselbe N-Protein-Aminosäuresequenz kodiert, wie die N-Gen-cDNA-Sequenz für den veslkulären Stomatitis-Virus-Stamm M2, M3 oder M4, wie in Figur 14 gezeigt.

28. Verwendung nach Anspruch 1, wobei das Virus eine P-Gen-Nukleinsäuresequenz aufweist, die für dieselbe P-Protein-Aminosäuresequenz kodiert, wie die P-Gen-cDNA-Sequenz für den vesikulären Stomatitis-Virus-Stamm M2, M3 oder M4, wie in Figur 16 gezeigt.

29. Verwendung nach Anspruch 1, wobei das Virus eine M-Gen-Nukleinsäuresequenz aufweist, die für dieselbe M-Proteln-Aminosäuresequenz kodiert, wie die M-Gen-cDNA-Sequenz für den vesikulären Stomatitis-Virus-Stamm M3 oder M4, wie in Figur 18 gezeigt.

30. Verwendung nach Anspruch 1, wobei das Virus eine G-Gen-Nukleinsäuresequenz aufweist, die für dieselbe G-Protein-Aminosäuresequenz kodiert, wie die G-Gen-cDNA-Sequenz für den vesikulären Stomatitis-Virus-Stamm M2, M3 oder M4, wie in Figur 20 gezeigt.

31. Verwendung nach Anspruch 1, wobei das Virus eine L-Gen-. Nukleinsäuresequenz aufweist, die für dieselbe L-Protein-Aminosäuresequenz kodiert, wie die L-Gen-cDNA-Sequenz für den vesikulären Stomatitis-Virus-Stamm M2 oder M4, wie in Figur 22 gezeigt.

32. Isoliertes Nukleinsäure-Molekül mit einer Sequenz, umfassend:
(a) eine Sequenz, die für ein Protein kodiert, umfassend die Aminosäuresequenz von Protein N des VSV-Stammes HR, wie in Figur 15 gezeigt; oder
(b) die N-Gen-cDNA-Sequenz eines VSV-Stammes, ausgewählt aus der Gruppe, bestehend aus HR, M2 und M3, wie in Figur 14 gezeigt oder
(c) eine RNA-Sequenz entsprechend (b); oder
(d) eine Sequenz komplementär zu (a), (b) oder (c).

33. Isoliertes Nukleinsäure-Molekül mit einer Sequenz, umfassend:
(a) eine Sequenz, die für ein Protein kodiert, umfassend die Aminosäuresequenz von Protein P des VSV-Stammes HR, wie in Figur 17 gezeigt; oder
(b) die P-Gen-cDNA-Sequenz eines VSV-Stammes, ausgewählt aus der Gruppe, bestehend aus HR und M3, wie in Figur 16 gezeigt; oder
(c) eine RNA-Sequenz entsprechend (b); oder
(d) eine Sequenz komplementär zu (a), (b) oder (c).

34. Isoliertes Nukleinsäure-Molekül mit einer Sequenz, umfassend:
(a) eine Sequenz, die für ein Protein kodiert, umfassend die Aminosäuresequenz von Protein M eines VSV-Stammes, ausgewählt aus der Gruppe, bestehend aus HR, M3 und M4, wie in Figur 19 gezeigt; oder
(b) die M-Gen-cDNA-Sequenz eines VSV-Stammes, ausgewählt aus der Gruppe, bestehend aus HR, M3 und M4, wie in Figur 18 gezeigt; oder
(c) eine RNA-Sequenz entsprechend (b); oder
(d) eine Sequenz komplementär zu (a), (b) oder (c).

35. Isoliertes Nukleinsäure-Molekül mit einer Sequenz, umfassend:
(a) eine Sequenz, die für ein Protein kodiert, umfassend die Aminosäuresequenz des Proteins G des VSV-Stammes M3, wie in Figur 21 gezeigt; oder
(b) eine G-Gen-cDNA-Sequenz des VSV-Stammes M3, wie in Figur 20 gezeigt; oder
(c) eine RNA-Sequenz entsprechend (b); oder
(d) eine Sequenz komplementär zu (a), (b) oder (c).

36. Isoliertes Nukleinsäure-Molekül mit einer Sequenz, umfassend:
(a) eine Sequenz, die für ein Protein kodiert, umfassend die Aminosäuresequenz des Proteins L des VSV-Stammes M4, wie in Figur 23 gezeigt; oder
(b) eine L-Gen-cDNA-Sequenz des VSV-Stammes M4, wie in Figur 12 gezeigt; oder
(c) eine RNA-Sequenz entsprechend (b); oder
(d) eine Sequenz komplementär zu (a), (b) oder (c).

## Revendications

1. Utilisation d'un virus pour la,fabrication d'un médicament pour réduire la viabilité d'une cellule tumorale, dans laquelle le virus est une souche atténuée du virus de la stomatite vésiculeuse et dans laquelle la cellule tumorale est une cellule de cancer hématopoïétique, de mélanome, de sarcome, de tumeur neuroendocrinienne, de carcinome du poumon ou de carcinome du côlon.

2. Utilisation selon la revendication 1, dans laquelle la cellule tumorale est une cellule de carcinome du poumon.

3. Utilisation selon la revendication 1, dans laquelle la cellule tumorale est une cellule de cancer hématopoïétique.

4. Utilisation selon la revendication 3, dans laquelle la cellule de cancer hématopoïétique est une cellule de leucémie, de lymphome ou de myélome.

5. Utilisation selon la revendication 4, dans laquelle la cellule de cancer hématopoïétique est une cellule de leucémie.

6. Utilisation selon la revendication 5, dans laquelle la leucémie est une leucémie myélogénique aiguë.

7. Utilisation selon la revendication 5, dans laquelle la leucémie est une leucémie myélogénique chronique.

8. Utilisation selon la revendication 5, dans laquelle la leucémie est une leucémie promyélocytaire.

9. Utilisation selon la revendication 5, dans laquelle la leucémie est une leucémie à cellules T.

10. Utilisation selon la revendication 4, dans laquelle la cellule de cancer hématopoïétique est un lymphome.

11. Utilisation selon la revendication 4, dans laquelle la cellule de cancer hématopoïétique est une cellule de myélome.

12. Utilisation selon la revendication 1, dans laquelle la cellule de tumeur est une cellule de sarcome.

13. Utilisation selon la revendication 12, dans laquelle le sarcome est un ostéosarcome.

14. Utilisation selon la revendication 12, dans laquelle le sarcome est un fibrosarcome.

15. Utilisation selon la revendication 1, dans laquelle la cellule tumorale est une cellule de tumeur neuroendocrinienne.

16. Utilisation selon la revendication 1, dans laquelle la cellule tumorale n'a sensiblement pas d'activité PKR.

17. Utilisation selon la revendication 1, dans laquelle la cellule tumorale est PKR-/- ; STAT1-/- ou PKR-/- et STAT1-/-.

18. Utilisation selon la revendication 5 comprenant en outre l'administration d'interféron à la cellule tumorale avant administration du VSV.

19. Utilisation selon la revendication 1, dans laquelle le virus est la souche M1 du virus de la stomatite vésiculeuse.

20. Utilisation selon la revendication 1, dans laquelle le virus est la souche M2 du virus de.la stomatite vésiculeuse.

21. Utilisation selon la revendication 1, dans laquelle le virus est la souche M3 du virus de la stomatite vésiculeuse.

22. Utilisation selon la revendication 1, dans laquelle le virus est la souche M4 du virus de la stomatite vésiculeuse.

23. Utilisation selon la revendication 1, dans laquelle le virus est la souche M5 du virus de la stomatite vésiculeuse.

24. Utilisation selon la revendication 1, dans laquelle la cellule tumorale se trouve dans un sujet mammifère et le virus est destiné à l'administration à la cellule tumorale par administration intraveineuse, intranasale, intrapéritonéale ou intra-tumorale au sujet.

25. Utilisation selon la revendication 24, dans laquelle le sujet mammifère est un humain ou un mammifère non humain.

26. Utilisation selon la revendication 24, dans laquelle le virus est contenu dans une lignée cellulaire infectée avec le virus et l'administration comprend l'administration de la lignée cellulaire infectée par le virus au sujet par une voie choisie parmi l'administration intra-tumorale, intraveineuse ou intrapéritonéale.

27. Utilisation selon la revendication 1, dans laquelle le virus a une séquence d'acide nucléique de gène N qui code la même séquence d'acides aminés de protéine N que la séquence d'ADNc du gène N pour la souche M2, M3 ou M4 du virus de la stomatite vésiculeuse, comme présentée sur la figure 14.

28. Utilisation selon la revendication 1, dans laquelle le virus a une séquence d'acide nucléique de gène P qui code la même séquence d'acides aminés de protéine P que la séquence d'ADNc du gène P pour la souche M2, M3 ou M4 du virus de la stomatite vésiculeuse, comme présentée sur la figure 16.

29. Utilisation selon la revendication 1, dans laquelle le virus a une séquence d'acide nucléique de gène M qui code la même séquence d'acides aminés de protéine M que la séquence d'ADNc du gène M pour la souche M2, M3 ou M4 du virus de la stomatite vésiculeuse, comme présentée sur la figure 18.

30. Utilisation selon la revendication 1, dans laquelle le virus a une séquence d'acide nucléique de gène G qui code la même séquence d'acides aminés de protéine G que la séquence d'ADNc du gène G pour la souche M2, M3 ou M4 du virus de la stomatite vésiculeuse, comme présentée sur la figure 20.

31. Utilisation selon la revendication 1, dans laquelle le virus a une séquence d'acide nucléique de gène L qui code la même séquence d'acides aminés de protéine L que la séquence d'ADNc de gène L de la souche M2 ou M4 du virus de la stomatite vésiculeuse, comme présentée sur la figure 22.

32. Molécule d'acide nucléique isolée ayant une séquence comprenant :
(a) une séquence codant une protéine comprenant la séquence d'acides aminés de la protéine N de la souche HR du VSV présentée sur la figure 15 ; ou
(b) la séquence d'ADNc du gène N d'une souche de VSV choisie dans le groupe comprenant HR, M2 et M3, présentée sur la figure 14 ; ou
(c) la séquence d'ARN correspondant à (b) ; ou
(d) une séquence complémentaire de (a), (b) ou (c).

33. Molécule d'acide nucléique isolée ayant une séquence comprenant :
(a) une séquence codant une protéine comprenant la séquence d'acides aminés de la protéine P de la souche HR du VSV présentée sur la figure 17 ; ou
(b) la séquence d'ADNc du gène P d'une souche de VSV choisie dans le groupe comprenant HR et M3 présentée sur la figure 16 ; ou
(c) la séquence d'ARN correspondant à (b) ; ou
(d) une séquence complémentaire de (a), (b) ou (c).

34. Molécule d'acide nucléique isolée ayant une séquence comprenant :
(a) une séquence codant une protéine comprenant la séquence d'acides aminés de la protéine M d'une souche du VSV choisie dans le groupe comprenant HR, M3 et M4, présentée sur la figure 19 ; ou
(b) la séquence d'ADNc du gène M d'une souche de VSV choisie dans le groupe comprenant HR, M3 et M4, présentée sur la figure 18 ; ou
(c) la séquence d'ARN correspondant à (b) ; ou
(d) une séquence complémentaire de (a), (b) ou (c).

35. Molécule d'acide nucléique isolée ayant une séquence comprenant :
(a) une séquence codant une protéine comprenant la séquence d'acides aminés de la protéine G de la souche M3 du VSV présentée sur la figure 21 ; ou
(b) la séquence d'ADNc du gène G de la souche M3 du VSV, présentée sur la figure 20 ; ou
(c) la séquence d'ARN correspondant à (b) ; ou
(d) une séquence complémentaire de (a), (b) ou (c).

36. Molécule d'acide nucléique isolée ayant une séquence comprenant :
(a) une séquence codant une protéine comprenant la séquence d'acides aminés de la protéine L de la souche M4 du VSV présentée sur la figure 23 ; ou
(b) la séquence d'ADNc du gène L de la souche M4 du VSV, présentée sur la figure 22 ; ou
(c) la séquence d'ARN correspondant à (b) ; ou
(d) une séquence complémentaire de (a), (b) ou (c).
